# EUROPÄISCHE PATENTSCHRIFT

(11) **EP 2 393 919 B1**
(45) Veröffentlichungstag und Bekanntmachung des Hinweises auf die Patenterteilung: **08.07.2015**
(21) Anmeldenummer: 10721938.8
(22) Anmeldetag: 05.02.2010
(51) Int. Cl.: C12N 5/073, C12N 15/85

(54) **NEUE PERMANENTE HUMANE ZELLLINIE**
NEW PERMANT HUMAN CELL LINE
NOUVELLE LIGNÉE CELLULAIRE HUMAINE PERMANENTE

(30) Priorität: 05.02.2009 DE 102009003439
(43) Veröffentlichungstag der Anmeldung: 14.12.2011
(60) Teilanmeldung: 15000822.5
(73) Patentinhaber: CEVEC Pharmaceuticals GmbH, 51105 Köln (DE)
(72) Erfinder: SCHIEDNER, Gudrun, 51105 Köln (DE); VOLPERS, Christoph, 65205 Wiesbaden (DE)
(74) Vertreter: Müller-Boré & Partner Patentanwälte PartG mbB
(86) Internationale Anmeldenummer: PCT/DE2010/075012
(87) Internationale Veröffentlichungsnummer: WO 2010/094280

(56) Entgegenhaltungen:
- US-A1- 2007 111 312
- US-B1- 6 558 948
- SCHIEDNER G ET AL: "EFFICIENT TRANSFORMATION OF PRIMARY HUMAN AMNIOCYTES BY E1 FUNCTIONS OF AD5: GENERATION OF NEW CELL LINES FOR ADENOVIRAL VECTOR PRODUCTION" HUMAN GENE THERAPY, MARY ANN LIEBERT, NEW YORK ,NY, US LNKD- DOI:10.1089/104303400750001417, Bd. 11, Nr. 15, 10. Oktober 2000 (2000-10-10), Seiten 2105-2116, XP000974022 ISSN: 1043-0342
- SCHIEDNER GUDRUN ET AL: "Efficient and reproducible generation of high-expressing, stable human cell lines without need for antibiotic selection" BMC BIOTECHNOLOGY, BIOMED CENTRAL LTD. LONDON, GB, Bd. 8, Nr. 1, 12. Februar 2008 (2008-02-12), Seite 13, XP021035682 ISSN: 1472-6750

### SEQUENCE LISTINTG

<110> CEVEC Pharmaceuticals GmbH
<120> Neue permanente humane Zelllinie
<130> CEV-014 PCT
<150> DE 10 2009 003 439.0
   <151> 2009-02-05
<160> 12
<170> PatentIn version 3.3
<210> 1
   <211> 4344
   <212> DNA
   <213> Artificial
<220>
   <223> 1-4344 Ad5
<400> 1
   catcatcaat aatatacctt attttggatt gaagccaata tgataatgag ggggtggagt      60
   ttgtgacgtg gcgcggggcg tgggaacggg gcgggtgacg tagtagtgtg gcggaagtgt     120
   gatgttgcaa gtgtggcgga acacatgtaa gcgacggatg tggcaaaagt gacgtttttg     180
   gtgtgcgccg gtgtacacag gaagtgacaa ttttcgcgcg gttttaggcg gatgttgtag     240
   taaatttggg cgtaaccgag taagatttgg ccattttcgc gggaaaactg aataagagga     300
   agtgaaatct gaataatttt gtgttactca tagcgcgtaa tatttgtcta gggccgcggg     360
   gactttgacc gtttacgtgg agactcgccc aggtgttttt ctcaggtgtt ttccgcgttc     420
   cgggtcaaag ttggcgtttt attattatag tcagctgacg tgtagtgtat ttatacccgg     480
   tgagttcctc aagaggccac tcttgagtgc cagcgagtag agttttctcc tccgagccgc     540
   tccgacaccg ggactgaaaa tgagacatat tatctgccac ggaggtgtta ttaccgaaga     600
   aatggccgcc agtcttttgg accagctgat cgaagaggta ctggctgata atcttccacc     660
   tcctagccat tttgaaccac ctacccttca cgaactgtat gatttagacg tgacggcccc     720
   cgaagatccc aacgaggagg cggtttcgca gatttttccc gactctgtaa tgttggcggt     780
   gcaggaaggg attgacttac tcacttttcc gccggcgccc ggttctccgg agccgcctca     840
   cctttcccgg cagcccgagc agccggagca gagagccttg ggtccggttt ctatgccaaa     900
   ccttgtaccg gaggtgatcg atcttacctg ccacgaggct ggctttccac ccagtgacga     960
   cgaggatgaa gagggtgagg agtttgtgtt agattatgtg gagcaccccg ggcacggttg    1020
   caggtcttgt cattatcacc ggaggaatac gggggaccca gatattatgt gttcgctttg    1080
   ctatatgagg acctgtggca tgtttgtcta cagtaagtga aaattatggg cagtgggtga 1140
   tagagtggtg ggtttggtgt ggtaattttt tttttaattt ttacagtttt gtggtttaaa 1200
   gaattttgta ttgtgatttt tttaaaaggt cctgtgtctg aacctgagcc tgagcccgag 1260
   ccagaaccgg agcctgcaag acctacccgc cgtcctaaaa tggcgcctgc tatcctgaga 1320
   cgcccgacat cacctgtgtc tagagaatgc aatagtagta cggatagctg tgactccggt 1380
   ccttctaaca cacctcctga gatacacccg gtggtcccgc tgtgccccat taaaccagtt 1440
   gccgtgagag ttggtgggcg tcgccaggct gtggaatgta tcgaggactt gcttaacgag 1500
   cctgggcaac ctttggactt gagctgtaaa cgccccaggc cataaggtgt aaacctgtga 1560
   ttgcgtgtgt ggttaacgcc tttgtttgct gaatgagttg atgtaagttt aataaagggt 1620
   gagataatgt ttaacttgca tggcgtgtta aatggggcgg ggcttaaagg gtatataatg 1680
   cgccgtgggc taatcttggt tacatctgac ctcatggagg cttgggagtg tttggaagat 1740
   ttttctgctg tgcgtaactt gctggaacag agctctaaca gtacctcttg gttttggagg 1800
   tttctgtggg gctcatccca ggcaaagtta gtctgcagaa ttaaggagga ttacaagtgg 1860
   gaatttgaag agcttttgaa atcctgtggt gagctgtttg attctttgaa tctgggtcac 1920
   caggcgcttt tccaagagaa ggtcatcaag actttggatt tttccacacc ggggcgcgct 1980
   gcggctgctg ttgctttttt gagttttata aaggataaat ggagcgaaga aacccatctg 2040
   agcggggggt acctgctgga ttttctggcc atgcatctgt ggagagcggt tgtgagacac 2100
   aagaatcgcc tgctactgtt gtcttccgtc cgcccggcga taataccgac ggaggagcag 2160
   cagcagcagc aggaggaagc caggcggcgg cggcaggagc agagcccatg gaacccgaga 2220
   gccggcctgg accctcggga atgaatgttg tacaggtggc tgaactgtat ccagaactga 2280
   gacgcatttt gacaattaca gaggatgggc aggggctaaa gggggtaaag agggagcggg 2340
   gggcttgtga ggctacagag gaggctagga atctagcttt tagcttaatg accagacacc 2400
   gtcctgagtg tattactttt caacagatca aggataattg cgctaatgag cttgatctgc 2460
   tggcgcagaa gtattccata gagcagctga ccacttactg gctgcagcca ggggatgatt 2520
   ttgaggaggc tattagggta tatgcaaagg tggcacttag gccagattgc aagtacaaga 2580
   tcagcaaact tgtaaatatc aggaattgtt gctacatttc tgggaacggg gccgaggtgg 2640
   agatagatac ggaggatagg gtggccttta gatgtagcat gataaatatg tggccggggg 2700
   tgcttggcat ggacggggtg gttattatga atgtaaggtt tactggcccc aattttagcg 2760
   gtacggtttt cctggccaat accaacctta tcctacacgg tgtaagcttc tatgggttta 2820
   acaatacctg tgtggaagcc tggaccgatg taagggttcg gggctgtgcc ttttactgct 2880
   gctggaaggg ggtggtgtgt cgccccaaaa gcagggcttc aattaagaaa tgcctctttg 2940
   aaaggtgtac cttgggtatc ctgtctgagg gtaactccag ggtgcgccac aatgtggcct 3000
   ccgactgtgg ttgcttcatg ctagtgaaaa gcgtggctgt gattaagcat aacatggtat 3060
   gtggcaactg cgaggacagg gcctctcaga tgctgacctg ctcggacggc aactgtcacc 3120
   tgctgaagac cattcacgta gccagccact ctcgcaaggc ctggccagtg tttgagcata 3180
   acatactgac ccgctgttcc ttgcatttgg gtaacaggag gggggtgttc ctaccttacc 3240
   aatgcaattt gagtcacact aagatattgc ttgagcccga gagcatgtcc aaggtgaacc 3300
   tgaacggggt gtttgacatg accatgaaga tctggaaggt gctgaggtac gatgagaccc 3360
   gcaccaggtg cagaccctgc gagtgtggcg gtaaacatat taggaaccag cctgtgatgc 3420
   tggatgtgac cgaggagctg aggcccgatc acttggtgct ggcctgcacc cgcgctgagt 3480
   ttggctctag cgatgaagat acagattgag gtactgaaat gtgtgggcgt ggcttaaggg 3540
   tgggaaagaa tatataaggt gggggtctta tgtagttttg tatctgtttt gcagcagccg 3600
   ccgccgccat gagcaccaac tcgtttgatg gaagcattgt gagctcatat ttgacaacgc 3660
   gcatgccccc atgggccggg gtgcgtcaga atgtgatggg ctccagcatt gatggtcgcc 3720
   ccgtcctgcc cgcaaactct actaccttga cctacgagac cgtgtctgga acgccgttgg 3780
   agactgcagc ctccgccgcc gcttcagccg ctgcagccac cgcccgcggg attgtgactg 3840
   actttgcttt cctgagcccg cttgcaagca gtgcagcttc ccgttcatcc gcccgcgatg 3900
   acaagttgac ggctcttttg gcacaattgg attctttgac ccgggaactt aatgtcgttt 3960
   ctcagcagct gttggatctg cgccagcagg tttctgccct gaaggcttcc tcccctccca 4020
   atgcggttta aaacataaat aaaaaaccag actctgtttg gatttggatc aagcaagtgt 4080
   cttgctgtct ttatttaggg gttttgcgcg cgcggtaggc ccgggaccag cggtctcggt 4140
   cgttgagggt cctgtgtatt ttttccagga cgtggtaaag gtgactctgg atgttcagat 4200
   acatgggcat aagcccgtct ctggggtgga ggtagcacca ctgcagagct tcatgctgcg 4260
   gggtggtgtt gtagatgatc cagtcgtagc aggagcgctg ggcgtggtgc ctaaaaatgt 4320
   ctttcagtag caagctgatt gcca 4344
<210> 2
   <211> 3018
   <212> DNA
   <213> Artificial
<220>
   <223> 505-3522 Ad5
<400> 2
   gagtgccagc gagtagagtt ttctcctccg agccgctccg acaccgggac tgaaaatgag 60
   acatattatc tgccacggag gtgttattac cgaagaaatg gccgccagtc ttttggacca 120
   gctgatcgaa gaggtactgg ctgataatct tccacctcct agccattttg aaccacctac 180
   ccttcacgaa ctgtatgatt tagacgtgac ggcccccgaa gatcccaacg aggaggcggt 240
   ttcgcagatt tttcccgact ctgtaatgtt ggcggtgcag gaagggattg acttactcac 300
   ttttccgccg gcgcccggtt ctccggagcc gcctcacctt tcccggcagc ccgagcagcc 360
   ggagcagaga gccttgggtc cggtttctat gccaaacctt gtaccggagg tgatcgatct 420
   tacctgccac gaggctggct ttccacccag tgacgacgag gatgaagagg gtgaggagtt 480
   tgtgttagat tatgtggagc accccgggca cggttgcagg tcttgtcatt atcaccggag 540
   gaatacgggg gacccagata ttatgtgttc gctttgctat atgaggacct gtggcatgtt 600
   tgtctacagt aagtgaaaat tatgggcagt gggtgataga gtggtgggtt tggtgtggta 660
   attttttttt taatttttac agttttgtgg tttaaagaat tttgtattgt gattttttta 720
   aaaggtcctg tgtctgaacc tgagcctgag cccgagccag aaccggagcc tgcaagacct 780
   acccgccgtc ctaaaatggc gcctgctatc ctgagacgcc cgacatcacc tgtgtctaga 840
   gaatgcaata gtagtacgga tagctgtgac tccggtcctt ctaacacacc tcctgagata 900
   cacccggtgg tcccgctgtg ccccattaaa ccagttgccg tgagagttgg tgggcgtcgc 960
   caggctgtgg aatgtatcga ggacttgctt aacgagcctg ggcaaccttt ggacttgagc 1020
   tgtaaacgcc ccaggccata aggtgtaaac ctgtgattgc gtgtgtggtt aacgcctttg 1080
   tttgctgaat gagttgatgt aagtttaata aagggtgaga taatgtttaa cttgcatggc 1140
   gtgttaaatg gggcggggct taaagggtat ataatgcgcc gtgggctaat cttggttaca 1200
   tctgacctca tggaggcttg ggagtgtttg gaagattttt ctgctgtgcg taacttgctg 1260
   gaacagagct ctaacagtac ctcttggttt tggaggtttc tgtggggctc atcccaggca 1320
   aagttagtct gcagaattaa ggaggattac aagtgggaat ttgaagagct tttgaaatcc 1380
   tgtggtgagc tgtttgattc tttgaatctg ggtcaccagg cgcttttcca agagaaggtc 1440
   atcaagactt tggatttttc cacaccgggg cgcgctgcgg ctgctgttgc ttttttgagt 1500
   tttataaagg ataaatggag cgaagaaacc catctgagcg gggggtacct gctggatttt 1560
   ctggccatgc atctgtggag agcggttgtg agacacaaga atcgcctgct actgttgtct 1620
   tccgtccgcc cggcgataat accgacggag gagcagcagc agcagcagga ggaagccagg 1680
   cggcggcggc aggagcagag cccatggaac ccgagagccg gcctggaccc tcgggaatga 1740
   atgttgtaca ggtggctgaa ctgtatccag aactgagacg cattttgaca attacagagg 1800
   atgggcaggg gctaaagggg gtaaagaggg agcggggggc ttgtgaggct acagaggagg 1860
   ctaggaatct agcttttagc ttaatgacca gacaccgtcc tgagtgtatt acttttcaac 1920
   agatcaagga taattgcgct aatgagcttg atctgctggc gcagaagtat tccatagagc 1980
   agctgaccac ttactggctg cagccagggg atgattttga ggaggctatt agggtatatg 2040
   caaaggtggc acttaggcca gattgcaagt acaagatcag caaacttgta aatatcagga 2100
   attgttgcta catttctggg aacggggccg aggtggagat agatacggag gatagggtgg 2160
   cctttagatg tagcatgata aatatgtggc cgggggtgct tggcatggac ggggtggtta 2220
   ttatgaatgt aaggtttact ggccccaatt ttagcggtac ggttttcctg gccaatacca 2280
   accttatcct acacggtgta agcttctatg ggtttaacaa tacctgtgtg gaagcctgga 2340
   ccgatgtaag ggttcggggc tgtgcctttt actgctgctg gaagggggtg gtgtgtcgcc 2400
   ccaaaagcag ggcttcaatt aagaaatgcc tctttgaaag gtgtaccttg ggtatcctgt 2460
   ctgagggtaa ctccagggtg cgccacaatg tggcctccga ctgtggttgc ttcatgctag 2520
   tgaaaagcgt ggctgtgatt aagcataaca tggtatgtgg caactgcgag gacagggcct 2580
   ctcagatgct gacctgctcg gacggcaact gtcacctgct gaagaccatt cacgtagcca 2640
   gccactctcg caaggcctgg ccagtgtttg agcataacat actgacccgc tgttccttgc 2700
   atttgggtaa caggaggggg gtgttcctac cttaccaatg caatttgagt cacactaaga 2760
   tattgcttga gcccgagagc atgtccaagg tgaacctgaa cggggtgttt gacatgacca 2820
   tgaagatctg gaaggtgctg aggtacgatg agacccgcac caggtgcaga ccctgcgagt 2880
   gtggcggtaa acatattagg aaccagcctg tgatgctgga tgtgaccgag gagctgaggc 2940
   ccgatcactt ggtgctggcc tgcacccgcg ctgagtttgg ctctagcgat gaagatacag 3000
   attgaggtac tgaaatgt 3018
<210> 3
   <211> 3575
   <212> DNA
   <213> Artificial
<220>
   <223> 505-4079 Ad5
<400> 3
   gagtgccagc gagtagagtt ttctcctccg agccgctccg acaccgggac tgaaaatgag 60
   acatattatc tgccacggag gtgttattac cgaagaaatg gccgccagtc ttttggacca 120
   gctgatcgaa gaggtactgg ctgataatct tccacctcct agccattttg aaccacctac 180
   ccttcacgaa ctgtatgatt tagacgtgac ggcccccgaa gatcccaacg aggaggcggt 240
   ttcgcagatt tttcccgact ctgtaatgtt ggcggtgcag gaagggattg acttactcac 300
   ttttccgccg gcgcccggtt ctccggagcc gcctcacctt tcccggcagc ccgagcagcc 360
   ggagcagaga gccttgggtc cggtttctat gccaaacctt gtaccggagg tgatcgatct 420
   tacctgccac gaggctggct ttccacccag tgacgacgag gatgaagagg gtgaggagtt 480
   tgtgttagat tatgtggagc accccgggca cggttgcagg tcttgtcatt atcaccggag 540
   gaatacgggg gacccagata ttatgtgttc gctttgctat atgaggacct gtggcatgtt 600
   tgtctacagt aagtgaaaat tatgggcagt gggtgataga gtggtgggtt tggtgtggta 660
   attttttttt taatttttac agttttgtgg tttaaagaat tttgtattgt gattttttta 720
   aaaggtcctg tgtctgaacc tgagcctgag cccgagccag aaccggagcc tgcaagacct 780
   acccgccgtc ctaaaatggc gcctgctatc ctgagacgcc cgacatcacc tgtgtctaga 840
   gaatgcaata gtagtacgga tagctgtgac tccggtcctt ctaacacacc tcctgagata 900
   cacccggtgg tcccgctgtg ccccattaaa ccagttgccg tgagagttgg tgggcgtcgc 960
   caggctgtgg aatgtatcga ggacttgctt aacgagcctg ggcaaccttt ggacttgagc 1020
   tgtaaacgcc ccaggccata aggtgtaaac ctgtgattgc gtgtgtggtt aacgcctttg 1080
   tttgctgaat gagttgatgt aagtttaata aagggtgaga taatgtttaa cttgcatggc 1140
   gtgttaaatg gggcggggct taaagggtat ataatgcgcc gtgggctaat cttggttaca 1200
   tctgacctca tggaggcttg ggagtgtttg gaagattttt ctgctgtgcg taacttgctg 1260
   gaacagagct ctaacagtac ctcttggttt tggaggtttc tgtggggctc atcccaggca 1320
   aagttagtct gcagaattaa ggaggattac aagtgggaat ttgaagagct tttgaaatcc 1380
   tgtggtgagc tgtttgattc tttgaatctg ggtcaccagg cgcttttcca agagaaggtc 1440
   atcaagactt tggatttttc cacaccgggg cgcgctgcgg ctgctgttgc ttttttgagt 1500
   tttataaagg ataaatggag cgaagaaacc catctgagcg gggggtacct gctggatttt 1560
   ctggccatgc atctgtggag agcggttgtg agacacaaga atcgcctgct actgttgtct 1620
   tccgtccgcc cggcgataat accgacggag gagcagcagc agcagcagga ggaagccagg 1680
   cggcggcggc aggagcagag cccatggaac ccgagagccg gcctggaccc tcgggaatga 1740
   atgttgtaca ggtggctgaa ctgtatccag aactgagacg cattttgaca attacagagg 1800
   atgggcaggg gctaaagggg gtaaagaggg agcggggggc ttgtgaggct acagaggagg 1860
   ctaggaatct agcttttagc ttaatgacca gacaccgtcc tgagtgtatt acttttcaac 1920
   agatcaagga taattgcgct aatgagcttg atctgctggc gcagaagtat tccatagagc 1980
   agctgaccac ttactggctg cagccagggg atgattttga ggaggctatt agggtatatg 2040
   caaaggtggc acttaggcca gattgcaagt acaagatcag caaacttgta aatatcagga 2100
   attgttgcta catttctggg aacggggccg aggtggagat agatacggag gatagggtgg 2160
   cctttagatg tagcatgata aatatgtggc cgggggtgct tggcatggac ggggtggtta 2220
   ttatgaatgt aaggtttact ggccccaatt ttagcggtac ggttttcctg gccaatacca 2280
   accttatcct acacggtgta agcttctatg ggtttaacaa tacctgtgtg gaagcctgga 2340
   ccgatgtaag ggttcggggc tgtgcctttt actgctgctg gaagggggtg gtgtgtcgcc 2400
   ccaaaagcag ggcttcaatt aagaaatgcc tctttgaaag gtgtaccttg ggtatcctgt 2460
   ctgagggtaa ctccagggtg cgccacaatg tggcctccga ctgtggttgc ttcatgctag 2520
   tgaaaagcgt ggctgtgatt aagcataaca tggtatgtgg caactgcgag gacagggcct 2580
   ctcagatgct gacctgctcg gacggcaact gtcacctgct gaagaccatt cacgtagcca 2640
   gccactctcg caaggcctgg ccagtgtttg agcataacat actgacccgc tgttccttgc 2700
   atttgggtaa caggaggggg gtgttcctac cttaccaatg caatttgagt cacactaaga 2760
   tattgcttga gcccgagagc atgtccaagg tgaacctgaa cggggtgttt gacatgacca 2820
   tgaagatctg gaaggtgctg aggtacgatg agacccgcac caggtgcaga ccctgcgagt 2880
   gtggcggtaa acatattagg aaccagcctg tgatgctgga tgtgaccgag gagctgaggc 2940
   ccgatcactt ggtgctggcc tgcacccgcg ctgagtttgg ctctagcgat gaagatacag 3000
   attgaggtac tgaaatgtgt gggcgtggct taagggtggg aaagaatata taaggtgggg 3060
   gtcttatgta gttttgtatc tgttttgcag cagccgccgc cgccatgagc accaactcgt 3120
   ttgatggaag cattgtgagc tcatatttga caacgcgcat gcccccatgg gccggggtgc 3180
   gtcagaatgt gatgggctcc agcattgatg gtcgccccgt cctgcccgca aactctacta 3240
   ccttgaccta cgagaccgtg tctggaacgc cgttggagac tgcagcctcc gccgccgctt 3300
   cagccgctgc agccaccgcc cgcgggattg tgactgactt tgctttcctg agcccgcttg 3360
   caagcagtgc agcttcccgt tcatccgccc gcgatgacaa gttgacggct cttttggcac 3420
   aattggattc tttgacccgg gaacttaatg tcgtttctca gcagctgttg gatctgcgcc 3480
   agcaggtttc tgccctgaag gcttcctccc ctcccaatgc ggtttaaaac ataaataaaa 3540
   aaccagactc tgtttggatt tggatcaagc aagtg 3575
<210> 4
   <211> 2499
   <212> DNA
   <213> Artificial
<220>
   <223> SV40 T-Antigen
<400> 4
   atcatggata aagttttaaa cagagaggaa tctttgcagc taatggacct tctaggtctt      60
   gaaaggagtg cctgggggaa tattcctctg atgagaaagg catatttaaa aaaatgcaag     120
   gagtttcatc ctgataaagg aggagatgaa gaaaaaatga agaaaatgaa tactctgtac     180
   aagaaaatgg aagatggagt aaaatatgct catcaacctg actttggagg cttctgggat     240
   gcaactgagg tatttgcttc ttccttaaat cctggtgttg atgcaatgta ctgcaaacaa     300
   tggcctgagt gtgcaaagaa aatgtctgct aactgcatat gcttgctgtg cttactgagg     360
   atgaagcatg aaaatagaaa attatacagg aaagatccac ttgtgtgggt tgattgctac     420
   tgcttcgatt gctttagaat gtggtttgga cttgatcttt gtgaaggaac cttacttctg     480
   tggtgtgaca taattggaca aactacctac agagatttaa agctctaagg taaatataaa     540
   atttttaagt gtataatgtg ttaaactact gattctaatt gtttgtgtat tttagattcc     600
   aacctatgga actgatgaat gggagcagtg gtggaatgcc tttaatgagg aaaacctgtt     660
   ttgctcagaa gaaatgccat ctagtgatga tgaggctact gctgactctc aacattctac     720
   tcctccaaaa aagaagagaa aggtagaaga ccccaaggac tttccttcag aattgctaag     780
   ttttttgagt catgctgtgt ttagtaatag aactcttgct tgctttgcta tttacaccac      840
   aaaggaaaaa gctgcactgc tatacaagaa aattatggaa aaatattctg taacctttat     900
   aagtaggcat aacagttata atcataacat actgtttttt cttactccac acaggcatag     960
   agtgtctgct attaataact atgctcaaaa attgtgtacc tttagctttt taatttgtaa    1020
   aggggttaat aaggaatatt tgatgtatag tgccttgact agagatccat tttctgttat     1080
   tgaggaaagt ttgccaggtg ggttaaagga gcatgatttt aatccagaag aagcagagga    1140
   aactaaacaa gtgtcctgga agcttgtaac agagtatgca atggaaacaa aatgtgatga    1200
   tgtgttgtta ttgcttggga tgtacttgga atttcagtac agttttgaaa tgtgtttaaa    1260
   atgtattaaa aaagaacagc ccagccacta taagtaccat gaaaagcatt atgcaaatgc    1320
   tgctatattt gctgacagca aaaaccaaaa aaccatatgc caacaggctg ttgatactgt    1380
   tttagctaaa aagcgggttg atagcctaca attaactaga gaacaaatgt taacaaacag    1440
   atttaatgat cttttggata ggatggatat aatgtttggt tctacaggct ctgctgacat    1500
   agaagaatgg atggctggag ttgcttggct acactgtttg ttgcccaaaa tggattcagt    1560
   ggtgtatgac tttttaaaat gcatggtgta caacattcct aaaaaaagat actggctgtt    1620
   taaaggacca attgatagtg gtaaaactac attagcagct gctttgcttg aattatgtgg    1680
   ggggaaagct ttaaatgtta atttgccctt ggacaggctg aactttgagc taggagtagc    1740
   tattgaccag tttttagtag tttttgagga tgtaaagggc actggagggg agtccagaga    1800
   tttgccttca ggtcagggaa ttaataacct ggacaattta agggattatt tggatggcag    1860
   tgttaaggta aacttagaaa agaaacacct aaataaaaga actcaaatat ttccccctgg    1920
   aatagtcacc atgaatgagt acagtgtgcc taaaacactg caggccagat ttgtaaaaca    1980
   aatagatttt aggcccaaag attatttaaa gcattgcctg gaacgcagtg agtttttgtt    2040
   agaaaagaga ataattcaaa gtggcattgc tttgcttctt atgttaattt ggtacagacc    2100
   tgtggctgag tttgctcaaa gtattcagag cagaattgtg gagtggaaag agagattgga    2160
   caaagagttt agtttgtcag tgtatcaaaa aatgaagttt aatgtggcta tgggaattgg    2220
   agttttagat tggctaagaa acagtgatga tgatgatgaa gacagccagg aaaatgctga    2280
   taaaaatgaa gatggtgggg agaagaacat ggaagactca gggcatgaaa caggcattga    2340
   ttcacagtcc caaggctcat ttcaggcccc tcagtcctca cagtctgttc atgatcataa    2400
   tcagccatac cacatttgta gaggttttac ttgctttaaa aaacctccca cacctccccc    2460
   tgaacctgaa acataaggat ccagcgatcc gcctgaatt                           2499
<210> 5
   <211> 523
   <212> DNA
   <213> Artificial
<220>
   <223> CMV-Promotor
<400> 5
   gttacataac ttacggtaaa tggcccgcct ggctgaccgc ccaacgaccc ccgcccattg      60
   acgtcaataa tgacgtatgt tcccatagta acgccaatag ggactttcca ttgacgtcaa     120
   tgggtggagt atttacggta aactgcccac ttggcagtac atcaagtgta tcatatgcca     180
   agtacgcccc ctattgacgt caatgacggt aaatggcccg cctggcatta tgcccagtac     240
   atgaccttat gggactttcc tacttggcag tacatctacg tattagtcat cgctattacc     300
   atggtgatgc ggttttggca gtacatcaat gggcgtggat agcggtttga ctcacgggga     360
   tttccaagtc tccaccccat tgacgtcaat gggagtttgt tttggcacca aaatcaacgg     420
   gactttccaa aatgtcgtaa caactccgcc ccattgacgc aaatgggcgg taggcgtgta     480
   cggtgggagg tctatataag cagagctcgt ttagtgaacc gtc                       523
<210> 6
   <211> 97
   <212> DNA
   <213> Artificial
<220>
   <223> SV40 SD/SA (intron)
<400> 6
   taagtttagt ctttttgtct tttatttcag gtcccggatc cggtggtggt gcaaatcaaa 60
   gaactgctcc tcagtggatg ttgcctttac ttctagg 97
<210> 7
   <211> 147
   <212> DNA
   <213> Artificial
<220>
   <223> SV40 polyA (T-Antigen)
<400> 7
   ggggatccag acatgataag atacattgat gagtttggac aaaccacaac tagaatgcag 60
   tgaaaaaaat gctttatttg tgaaatttgt gatgctattg ctttatttgt aaccattata 120
   agctgcaata aacaagttaa caacaac 147
<210> 8
   <211> 1926
   <212> DNA
   <213> Artificial
<220>
   <223> EBNA-1
<400> 8
   atgtctgacg aggggccagg tacaggacct ggaaatggcc taggagagaa gggagacaca 60
   tctggaccag aaggctccgg cggcagtgga cctcaaagaa gagggggtga taaccatgga 120
   cgaggacggg gaagaggacg aggacgagga ggcggaagac caggagcccc gggcggctca 180
   ggatcagggc caagacatag agatggtgtc cggagacccc aaaaacgtcc aagttgcatt 240
   ggctgcaaag ggacccacgg tggaacagga gcaggagcag gagcgggagg ggcaggagca 300
   ggaggggcag gagcaggagg aggggcagga gcaggaggag gggcaggagg ggcaggaggg 360
   gcaggagggg caggagcagg aggaggggca ggagcaggag gaggggcagg aggggcagga 420
   ggggcaggag caggaggagg ggcaggagca ggaggagggg caggaggggc aggagcagga 480
   ggaggggcag gaggggcagg aggggcagga gcaggaggag gggcaggagc aggaggaggg 540
   gcaggagggg caggagcagg aggaggggca ggaggggcag gaggggcagg agcaggagga 600
   ggggcaggag caggaggggc aggaggggca ggaggggcag gagcaggagg ggcaggagca 660
   ggaggagggg caggaggggc aggaggggca ggagcaggag gggcaggagc aggaggggca 720
   ggagcaggag gggcaggagc aggaggggca ggaggggcag gagcaggagg ggcaggaggg 780
   gcaggagcag gaggggcagg aggggcagga gcaggaggag gggcaggagg ggcaggagca 840
   ggaggagggg caggaggggc aggagcagga ggggcaggag gggcaggagc aggaggggca 900
   ggaggggcag gagcaggagg ggcaggaggg gcaggagcag gaggaggggc aggagcagga 960
   ggggcaggag caggaggtgg aggccggggt cgaggaggca gtggaggccg gggtcgagga 1020
   ggtagtggag gccggggtcg aggaggtagt ggaggccgcc ggggtagagg acgtgaaaga 1080
   gccagggggg gaagtcgtga aagagccagg gggagaggtc gtggacgtgg agaaaagagg 1140
   cccaggagtc ccagtagtca gtcatcatca tccgggtctc caccgcgcag gccccctcca 1200
   ggtagaaggc catttttcca ccctgtaggg gaagccgatt attttgaata ccaccaagaa 1260
   ggtggcccag atggtgagcc tgacgtgccc ccgggagcga tagagcaggg ccccgcagat 1320
   gacccaggag aaggcccaag cactggaccc cggggtcagg gtgatggagg caggcgcaaa 1380
   aaaggagggt ggtttggaaa gcatcgtggt caaggaggtt ccaacccgaa atttgagaac 1440
   attgcagaag gtttaagagc tctcctggct aggagtcacg tagaaaggac taccgacgaa 1500
   ggaacttggg tcgccggtgt gttcgtatat ggaggtagta agacctccct ttacaaccta 1560
   aggcgaggaa ctgcccttgc tattccacaa tgtcgtctta caccattgag tcgtctcccc 1620
   tttggaatgg cccctggacc cggcccacaa cctggcccgc taagggagtc cattgtctgt 1680
   tatttcatgg tctttttaca aactcatata tttgctgagg ttttgaagga tgcgattaag 1740
   gaccttgtta tgacaaagcc cgctcctacc tgcaatatca gggtgactgt gtgcagcttt 1800
   gacgatggag tagatttgcc tccctggttt ccacctatgg tggaaggggc tgccgcggag 1860
   ggtgatgacg gagatgacgg agatgaagga ggtgatggag atgagggtga ggaagggcag 1920
   gagtga 1926
<210> 9
   <211> 29
   <212> DNA
   <213> Artificial
<220>
   <223> Primer
<400> 9
   ccggaattct ttgcaaaagc ctaggcctc 29
<210> 10
   <211> 29
   <212> DNA
   <213> Artificial
<220>
   <223> Primer
<400> 10
   ccggaattct gaggcggaaa gaaccagct 29
<210> 11
   <211> 359
   <212> DNA
   <213> Artificial
<220>
   <223> SV40 ori
<400> 11
   tttgcaaaag cctaggcctc caaaaaagcc tcctcactac ttctggaata gctcagaggc 60
   cgaggcggcc tcggcctctg cataaataaa aaaaattagt cagccatggg gcggagaatg 120
   ggcggaactg ggcggagtta ggggcgggat gggcggagtt aggggcggga ctatggttgc 180
   tgactaattg agatgcatgc tttgcatact tctgcctgct ggggagcctg gggactttcc 240
   acacctggtt gctgactaat tgagatgcat gctttgcata cttctgcctg ctggggagcc 300
   tggggacttt ccacacccta actgacacac attccacagc tggttctttc cgcctcaca 359
<210> 12
   <211> 1378
   <212> DNA
   <213> Artificial
<220>
   <223> hAAT
<400> 12
   attctgcagg gggggggggg ggctgggaca gtgaatcgac aatgccgtct tctgtctcgt 60
   ggggcatcct cctgctggca ggcctgtgct gcctggtccc tgtctccctg gctgaggatc 120
   cccagggaga tgctgcccag aagacagata catcccacca tgatcaggat cacccaacct 180
   tcaacaagat cacccccaac ctggctgagt tcgccttcag cctataccgc cagctggcac 240
   accagtccaa cagcaccaat atcttcttct ccccagtgag catcgctaca gcctttgcaa 300
   tgctctccct ggggaccaag gctgacactc acgatgaaat cctggagggc ctgaatttca 360
   acctcacgga gattccggag gctcagatcc atgaaggctt ccaggaactc ctccgtaccc 420
   tcaaccagcc agacagccag ctccagctga ccaccggcaa tggcctgttc ctcagcgagg 480
   gcctgaagct agtggataag tttttggagg atgttaaaaa gttgtaccac tcagaagcct 540
   tcactgtcaa cttcggggac accgaagagg ccaagaaaca gatcaacgat tacgtggaga 600
   agggtactca agggaaaatt gtggatttgg tcaaggagct tgacagagac acagtttttg 660
   ctctggtgaa ttacatcttc tttaaaggca aatgggagag accctttgaa gtcaaggaca 720
   ccgaggaaga ggacttccac gtggaccagg tgaccaccgt gaaggtgcct atgatgaagc 780
   gtttaggcat gtttaacatc cagcactgta agaagctgtc cagctgggtg ctgctgatga 840
   aatacctggg caatgccacc gccatcttct tcctgcctga tgaggggaaa ctacagcacc 900
   tggaaaatga actcacccac gatatcatca ccaagttcct ggaaaatgaa gacagaaggt 960
   ctgccagctt acatttaccc aaactgtcca ttactggaac ctatgatctg aagagcgtcc 1020
   tgggtcaact gggcatcact aaggtcttca gcaatggggc tgacctctcc ggggtcacag 1080
   aggaggcacc cctgaagctc tccaaggccg tgcataaggc tgtgctgacc atcgacgaga 1140
   aagggactga agctgctggg gccatgtttt tagaggccat acccatgtct atcccccccg 1200
   aggtcaagtt caacaaaccc tttgtcttct taatgattga acaaaatacc aagtctcccc 1260
   tcttcatggg aaaagtggtg aatcccaccc aaaaataact gcctctcgct cctcaacccc 1320
   tcccctccat ccctggcccc ctccctggat gacattaaag aagggttgag ctggattg 1378

## Beschreibung

Die vorliegende Erfindung betrifft ein Verfahren zur Herstellung einer permanenten humanen Amniozyten-Zelllinie, umfassend das Transfizieren primärer humaner Amniozyten-Zellen mit einem Nukleinsäuremolekül umfassend die Nukleinsäuresequenz kodierend für die adenoviralen Genprodukte E1A und E1B und das anschließende Transfizieren mit einem Nukleinsäuremolekül umfassend eine Nukleinsäuresequenz kodierend für das große T-Antigen von SV40 oder das Epstein-Barr-Virus (EBV) Nuclear Antigen-1 (EBNA-1).

Neben Bakterien, Hefen und Pflanzenzellen werden insbesondere tierische Zellen zur Produktion rekombinanter Polypeptide oder Proteine verwendet. Etwa 60-70% aller therapeutischen Proteine werden heute in Säugerzellen produziert (Wurm, Nat. Biotechnology 22, 1393-1398, 2004). Die Produktion von rekombinanten Polypeptiden oder Proteinen für therapeutische, diagnostische oder technische Zwecke in Zellkultur, d.h. in vitro, kann grundsätzlich auf zwei unterschiedliche Weisen erfolgen. In stabilen, dauerhaft oder permanent etablierten Zelllinien ist die Nukleinsäure, die das gewünschte Polypeptid oder Protein kodiert, in mindestens einer Kopie in die chromosomale DNA der Zelle integriert und wird bei der Zellteilung mit dem zellulären Chromosomensatz an die Tochterzellen weitergegeben (sog. stabile Expression in Produktionszelllinien). Zur Herstellung dieser stabilen Produktionszelllinien ist es erforderlich, dass mindestens eine der in die Zelle durch Transfektion eingebrachten Nukleinsäuren eine Genfunktion trägt, die der Zelle einen Selektionsvorteil beim Wachstum in der Zellkultur verschafft. Die Nukleinsäure, die eine solche Genfunktion enthält, muss nicht notwendigerweise auf dem gleichen Molekül wie die Expressionskassette für das gewünschte Polypeptid oder Protein vorhanden sein. Die Genfunktion ist entweder ein Resistenzgen gegen Antibiotika oder Chemotherapeutika im Medium (z.B. häufig bei Säugerzellen verwendet; Wurm, Nat. Biotechnology 22, 1393-1398, 2004), ein Gen, dessen Genprodukt einen defizienten Stoffwechselweg komplementiert (z.B. bei Hefezellen verwendet), oder eine transformierende Genfunktion (für humane Fruchtwasserzellen gezeigt; Schiedner et al., BMC Biotechnology 8, 13, 2008). Auf diese Weise wird gewährleistet, dass solche Zellen, bei denen eine stabile Integration der transfizierten Nukleinsäure in die chromosomale DNA der Zelle erfolgt ist und das Genprodukt hergestellt wird, die anderen Zellen ohne derartige Integration überwachsen und selektiert werden können. Bei der Gewinnung der Produktionszellen wird durch Transfektion in die sog. Wirts-Zelllinie zum einen die Nukleinsäure, die das rekombinante Polypeptid kodiert (das sog. Transgen), mit den notwendigen transkriptionellen Regulationselementen transferiert, zum anderen eine zweite Expressionskassette mit einem Gen, das einen Selektionsmarker kodiert und dessen Genprodukt der Zelle einen bestimmten Selektionsvorteil verschafft. Wenige Tage nach dem Gentransfer, während denen die Zellen z.B. in einem Kulturmedium ohne Selektionsreagenz kultiviert werden, wird dem Medium ein geeignetes Selektionsreagenz zugesetzt. In Gegenwart des Selektionsreagenz überleben und wachsen nur die Zellen, die die zur Transfektion benutzten Nukleinsäuren aufgenommen haben und den Selektionsmarker exprimieren. Häufig benutzte Selektionsmarker sind das Neomycin-Resistenzgen, das Hygromycin-Resistenzgen und die Dihydrofolat-Reduktase (DHFR)(Wurm, Nat. Biotechnology 22, 1393-1398, 2004; Wurm und Jordan, 309-333 in: Makrides (Hrsg.), Gene Transfer and Expression in Mammalian Cells, Elsevier, Amsterdam, 2003). Die Selektion erfolgt entsprechend in Kulturmedium mit den Selektionsreagenzien wie den Antibiotika Neomycin oder Hygromycin bzw. dem synthetischen Glukokortikoid Methotrexat. Zellen mit dem Selektionsmarker und dem Transgen, die den Selektionsprozess überleben und proliferieren (sog. Transformanten), werden in der Regel anschließend vereinzelt (kloniert), um sicherzustellen, dass alle Zellen in der Kultur genetisch identisch sind und um die gewünschten Produktionszelllinien mit der besten Produktionsrate von weniger gut produzierenden Zelllinien zu trennen.

Bei der sog. transienten Expression wird dagegen die durch Transfektion in die Zelle eingebrachte Nukleinsäure, die das gewünschte Polypeptid oder Protein kodiert, nicht in die chromosomale DNA der Zelle integriert bzw. nicht auf dieses Ereignis selektioniert, und damit wird die eingebrachte Nukleinsäure in der Regel im Laufe der Zellteilungen während des Wachstums der Kultur ausverdünnt und geht verloren, was die vorübergehende, transiente Natur dieses Expressionsverfahrens bedingt. Die Selektion stabiler Produktionszelllinien mit guter Expressionseffizienz dauert einige Monate und verursacht beträchtliche Kosten. Durch transiente Expression können dagegen durchaus Milligramm-Mengen des gewünschten Polypeptids oder Proteins innerhalb einiger Tage hergestellt werden. Geschwindigkeit und Kosten sind wesentliche Faktoren bei der industriellen Entwicklung von biopharmazeutischen und diagnostischen Produkten. Die transiente Expression von Proteinen in kleineren Mengen oder von verschiedenen Varianten eines Proteins wird deshalb außer in der Grundlagenforschung für die frühe explorative und präklinische Entwicklung durchgeführt, z.B. bei der Target-Identifizierung, Assay-Entwicklung, biochemischen Charakterisierung der Genprodukte, für die Toxikologie und pharmakokinetische sowie pharmakodynamische Untersuchungen (Baldi et al., Biotechnol. Lett. 29, 677-684, 2007; Pham et al., Molecular Biotechnology 34, 225-237, 2006). Die industrielle Herstellung von Proteinen im Gramm- bis Kilogramm-Maßstab für die Durchführung größerer klinischer Studien und die Marktversorgung erfolgt dagegen mit Hilfe stabiler Produktionszelllinien.

In EP 1948789 wird beispielsweise ein Verfahren zur Herstellung einer permanenten humanen Amniozyten-Zelllinie durch Transfektion eines Zell-transformierenden Faktors ohne die Verwendung eines Selektionsmarkers beschrieben.

Sezernierte, membranständige und intrazelluläre Proteine konnten bisher durch transiente Genexpression hergestellt werden. Säugerzellen sind für viele komplexe Proteine die derzeit gebräuchlichen Expressionssysteme, insbesondere wenn die Proteine für therapeutische Zwecke eingesetzt werden sollen, da prokaryotische und niedrige eukaryotische Zellsysteme (z.B. Hefen) hinsichtlich posttranslationaler Modifikationen deutlich benachteiligt sind. Für die transiente Proteinexpression wurden bisher im wesentlichen vier Säugerzelllinien verwendet: COS-1 bzw. COS-7-Zellen, die von der CV-1-Affennierenzelllinie der afrikanischen Grünen Meerkatze abstammen; BHK-Zellen, die von neonatalen Hamster-Nierenzellen abstammen; CHO-Zellen, die von Ovarialzellen des Chinesischen Hamsters abstammen; und HEK293-Zellen, eine humane embryonale Nierenzelllinie mit neuronalen Charakteristika (Pham et al., Molecular Biotechnology 34, 225-237, 2006; Wurm et Bernard, Current Opinion in Biotechnology 10, 156-159, 1999). Neben der Verwendung von viralen Expressionsvektoren wie Semliki-Forest-Virus oder Adenovirus, die effizient aber aufwendig und mit hohen Sicherheitsanforderungen verbunden und deshalb wenig verbreitet sind, basiert die transiente Expression in diesen Säugerzelllinien in der Regel auf der Transfektion eines Plasmidvektors, der die Expressionskassette mit der kodierenden Sequenz für das gewünschte Genprodukt enthält. Für den DNA-Transfer in kultivierte Säugerzellen wurde eine Vielzahl von physikalischen und chemischen Methoden entwickelt. Zu den physikalischen Gentransfermethoden gehören die Elektroporation, die Nukleofektion und die Mikroinjektion. Bei den chemischen Transfektionsverfahren bedient man sich anorganischer Substanzen (z.B. Calciumphosphat/DNA-Copräzipitation), kationischer Polymere (z.B. Polyethylenimin, DEAE/Dextran-Verfahren) oder kationischer Lipide (sog. Lipofektion). Calciumphosphat und Polyethylenimin sind die am häufigsten für den Nukleinsäuretransfer in größerem Maßstab (bis einige Liter) verwendeten Transfektionsreagentien (Baldi et al., Biotechnol. Lett. 29, 677-684,2007).

Die beschriebenen Verfahren zur transienten Expression von Polypeptiden und Proteinen auf der Basis lange bekannter Zelllinien haben in verschiedener Hinsicht Nachteile. Ein Problem im Zusammenhang mit transienten Verfahren sind die geringen Expressionsleistungen. Zur Verbesserung der zellulären Expressionsausbeuten wurden verschiedene genetische Systeme benutzt, um die Anzahl der Genkopien pro Zelle mit Hilfe episomaler Replikation der eingebrachten Nukleinsäure zu erhöhen. COS-Zellen exprimieren das große T-Antigen von Simian Virus 40 (SV40), ein Replikationsfaktor, der eine episomale Replikation von Plasmiden, die einen SV40-Replikationsursprung (SV40 ori) tragen, auf hohe Kopienzahl bewirkt. Initiales Ereignis dieser Replikation ist die Bindung des T-Antigens an den SV40-Replikationsursprung (SV40 ori), wodurch zelluläre Replikationsfaktoren an den DNA/T-Antigen-Komplex rekrutiert und eine Replikation mittels zellulärer DNA-Polymerase eingeleitet wird. Von der HEK293-Zelllinie, die vor etwa 30 Jahren durch Transformation humaner ernbryonaler Nierenzellen mit gescherter Adenovirus-Typ 5-DNA generiert wurde und die gut transfizierbar ist, wurden zwei genetische Varianten beschrieben, die ebenfalls das große T-Antigen von SV40 (HEK293T) bzw. das Epstein-Barr-Virus (EBV) Nuclear Antigen-1 (EBNA-1) exprimieren (HEK293E oder 293EBNA-1). Diese Zelllinien sollen eine episomale Replikation oder Amplifikation von Plasmiden gewährleisten, die einen SV40-ori bzw. einen EBV-oriP enthalten. Der Replikationsfaktor EBNA-1 wechselwirkt im letzteren Falle mit dem EBV-Replikationsursprung oriP. Zumindest für HEK293E-Zellen ist eine Erhöhung der Expressionsausbeuten bei Verwendung von oriP-enthaltenden Expressionsplasmiden nachgewiesen. Im Gegensatz zur Verwendung von EBNA-1 in Kombination mit dem Replikationsursprung oriP deuten manche Studien daraufhin, dass keine starke Replikation von SV40-ori-haltigen Plasmiden in HEK293T-Zellen erfolgt (Durocher et al., Nucleic Acid Research 30, e9, 2002). Von CHO-Zellen wurde eine stabile Variante generiert, die das große T-Antigen (LT) von Polyomavirus exprimiert (Epi-CHO) und in Kombination mit einem Plasmid eingesetzt werden kann, das den Polyomavirus-Replikationsursprung (PyOri) trägt (Kunaparaju et al., Biotechnology and Bioengineering 91, 670-677, 2005). Mit solchen Säugerzell-Systemen werden im Durchschnitt bei der transienten Expression rekombinanter Proteine Ausbeuten von etwa 10-20 mg/Liter erzielt (Baldi et al., Biotechnol. Lett. 29, 677-684, 2007). Mit stabilen, permanenten Produktionszelllinien sind dagegen durchaus Ausbeuten im Bereich von mehreren Gramm pro Liter üblich, allerdings wie beschrieben mit ganz erheblich höherem Zeit- und Kostenaufwand.

Ein weiterer Nachteil der bisher für die rekombinante Proteinexpression verwendeten Zellsysteme besteht darin, dass sich manche Zelllinien auf Grund guter Transfizierbarkeit und der Fähigkeit zur episomalen Plasmid-Amplifikation zwar für die transiente Expression eignen (z.B. HEK293T oder HEK293E-Zellen), aber andere Zelllinien wegen ihrer Kultivierungseigenschaften und Ausbeuten bevorzugt für die Herstellung stabiler Zelllinien verwendet werden (z.B. CHO-Zellen). Da sich Zellsysteme in verschiedenen Aspekten der posttranslationalen Modifikation aber deutlich unterscheiden, können nach transienter Expression (meist in der frühen Entwicklungsphase proteintherapeutischer Produkte) in einem best. Zellsystem gewonnene Daten zu Struktur und Funktion der Genprodukte nur sehr eingeschränkt auf die Struktur und Funktion dieser Genprodukte nach Expression in stabilen Zelllinien eines anderen Zellsystems (meist in der späteren Entwicklungsphase, für klinische Studien und Marktversorgung) übertragen werden. Bei vielen therapeutischen Produktkandidaten sind posttranslationale Modifikationen, wie z.B. Glykosylierung, Phosphorylierung, Carboxylierung, Palmitylierung oder spezifische Spaltungen, für unterschiedliche Eigenschaften der Expressionsprodukte von großer Bedeutung. Sie können wesentlichen Einfluss auf die Aktivität, Löslichkeit, Halbwertszeit, Stabilität oder Immunogenität haben. Deshalb spielen humane Zellsysteme für die Herstellung therapeutischer Proteine eine zunehmende Rolle; nur humane Zellen als Produktionsstätten gewährleisten eine authentische, humane Modifizierung der Expressionsprodukte und reduzieren damit das Risiko beeinträchtigter Produktqualitäten oder unerwünschter Nebenwirkungen. So ist beispielsweise für rekombinantes Erythropoietin, das humantherapeutisch eingesetzt wird, bekannt, dass in CHO-Zellen hergestelltes Protein (Epoetin Alpha) N-Glycolyl-Neuraminsäure-Reste in seinen Kohlenhydrat-Seitenketten aufweist, während das in humanen Zellen hergestellte Protein (Epoetin Delta) - genauso wie das natürliche humane Erythropoietin - keine solchen Zuckerreste enthält. Vor dem Hintergrund, dass der Mensch nachweislich zirkulierende Antikörper gegen diese "fremden" Zuckerstrukturen bildet, erscheint der Einsatz eines humanen Expressionssystems vorteilhaft (Varki, Am. J. Phys. Anthropol. 33, 54-69, 2001). Zur Zeit ist kein humanes Zellsystem verfügbar, das sich für die transiente Expression und die Herstellung stabiler Produktionszelllinien gleichermaßen gut eignet und damit ein reproduzierbares Produktprofil über die gesamte Entwicklung eines proteinbasierten Therapeutikums gewährleistet. Humane Zellen sind für die Herstellung humaner Biotherapeutika besonders gut geeignet, da sie komplexe Polypeptide - im Gegensatz zu anderen Säugerzellen oder tierischen Zellen - mit authentischem posttranslationalem Modifikationsmuster exprimieren. Das Glykosylierungsmuster komplexer rekombinanter Proteine, also die Struktur und Anordnung der Zuckerreste im Molekül, wird bei einer Herstellung in humanen Zellen wesentlich besser das Muster des authentischen humanen Polypeptids reproduzieren als bei einer Herstellung in nicht-humanen Produktionssystemen. Dieses Glykosylierungsmuster ist häufig für wichtige Eigenschaften des Polypeptids wie biologische Aktivität, Stabilität, Löslichkeit und Immunogenität von entscheidender Bedeutung.

Daher liegt der Erfindung die Aufgabe zu Grunde, ein Verfahren zur Herstellung einer permanenten humanen Amniozyten-Zelllinie bereitzustellen, das sich für die transiente Expression von Polypeptiden und die Herstellung stabiler Produktionszelllinien gleichermaßen gut eignet.

Die Aufgabe wird durch den in den Patentansprüchen definierten Gegenstand gelöst.

Die nachfolgenden Figuren erläutern die Erfindung.

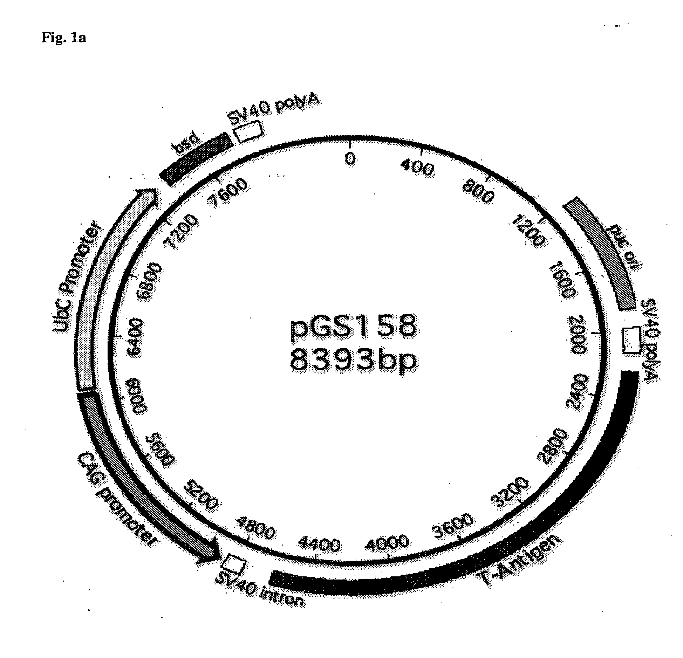

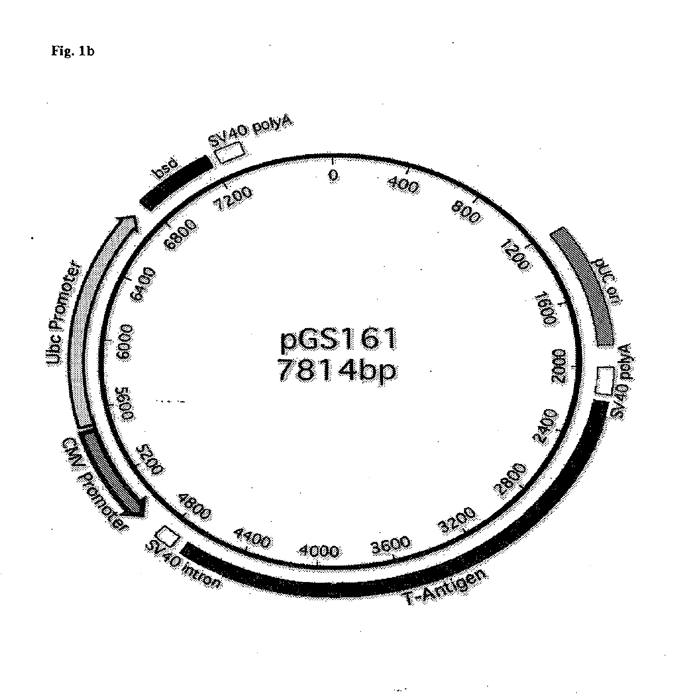

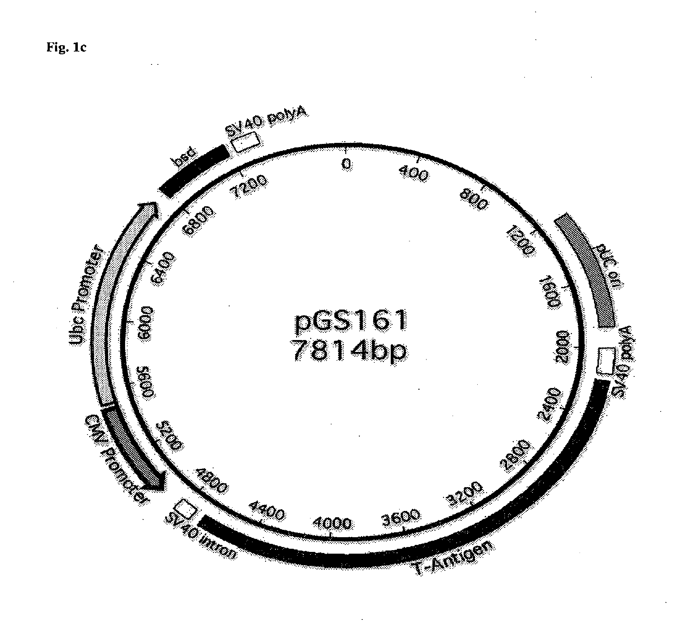

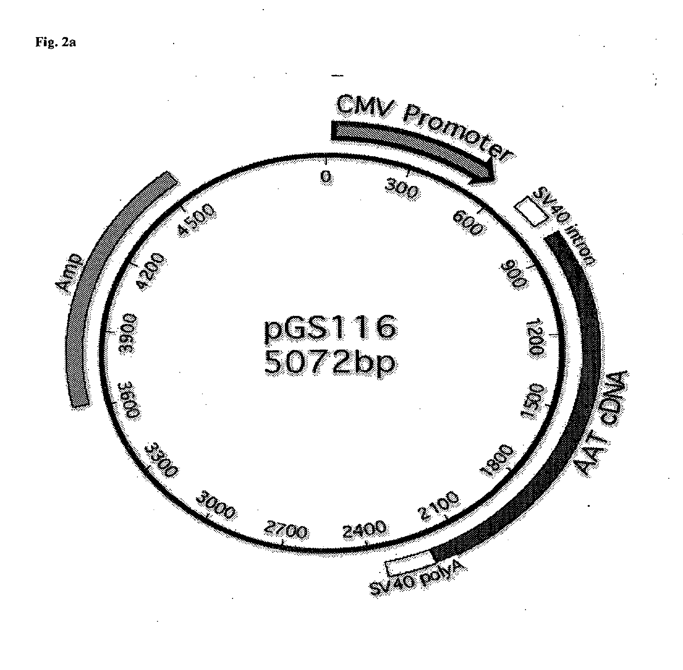

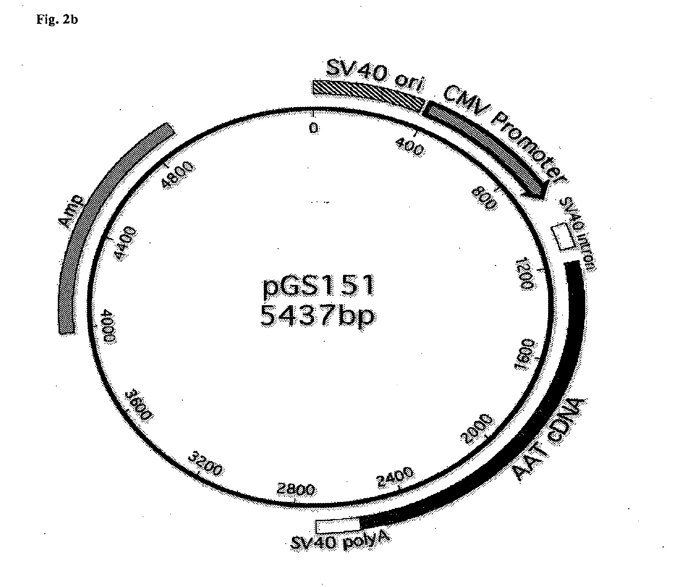

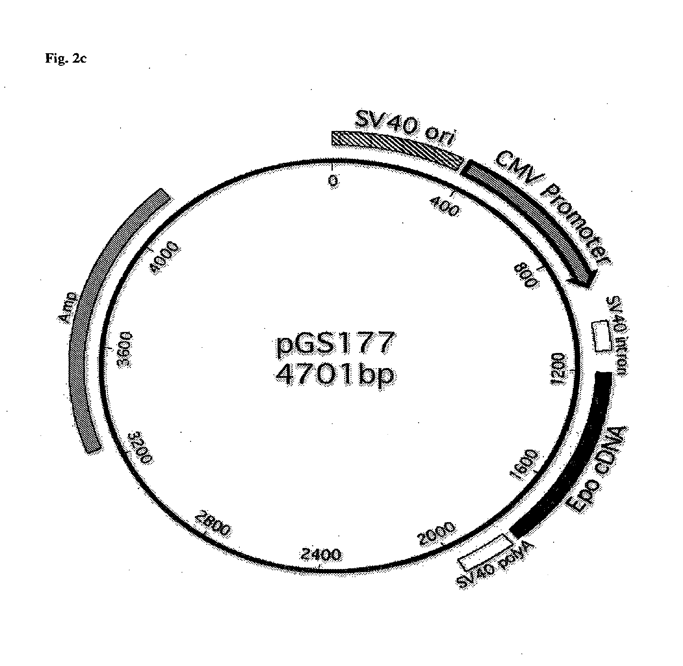

Fig. 1 zeigt schematisch den Aufbau der Plasmide zur permanenten Expression von T-Antigen. In pGS158 (Fig. 1a) wird T-Antigen unter der Kontrolle des humanen CAG-Promotors (ein Hybridpromotor aus dem Immediate Early-Enhancer des humanen Cytomegalievirus und einem modifizierten Huhn β-Aktin Promotor mit erstem Intron) (Niwa et al., Gene 108:193-199, 1991) exprimiert, in pGS159 (Fig. 1b) unter der Kontrolle des RSV-(Rous Sarkoma Virus-) Promotors (Makrides, 9-26 in: Makrides (Hrsg.), Gene Transfer and Expression in Mammalian Cells, Elsevier, Amsterdam, 2003) und in pGS161 (Fig. lc) unter Kontrolle des humanen CMV- (Cytomegalievirus-) Promotors (Makrides, 9-26 in: Makrides (Hrsg.), Gene Transfer and Expression in Mammalian Cells, Elsevier, Amsterdam, 2003). Fig. 2 zeigt schematisch den Aufbau der Plasmide zur transienten Expression des humanen Alpha1-Antitrypsins (hAAT) bzw. humanen Erythropoietins (Epo), jeweils unter der Kontrolle des humanen CMV-Promotors. Plasmid pGS116 (Fig. 2a) und pGS151 (Fig. 2b) haben identische Expressionskassetten für hAAT, pGS151 enthält zusätzlich den Startpunkt der DNA-Replikation von Simian Virus 40 (SV40 ori). pGS177 enthält ebenfalls zusätzlich zur Epo-Expressionskassette den SV40 ori.

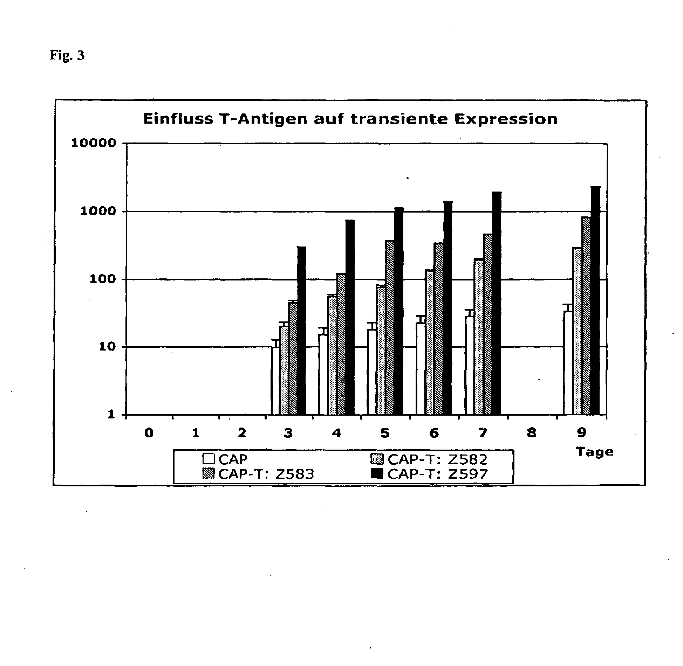

Fig. 3 zeigt schematisch die Menge an transient im Kulturüberstand exprimiertem hAAT in verschiedenen T-Antigen-exprimierenden Amniozyten-Zelllinien (CAP-T Z582, Z583 und Z597) im Vergleich zur parentalen Amniozyten-Zelllinie (CAP) ohne T-Antigenexpression. In Z582 wird das T-Antigen unter Kontrolle des CAG-Promotors (Niwa et al., Gene 108:193-199, 1991) exprimiert, in Z583 unter Kontrolle des RSV-(Rous Sarkoma Virus-) Promotors (Makrides, 9-26 in: Makrides (Hrsg.), Gene Transfer and Expression in Mammalian Cells, Elsevier, Amsterdam, 2003) und in Z597 unter Kontrolle des CMV-(Cytomegalie) Promotors (Makrides, 9-26 in: Makrides (Hrsg.), Gene Transfer and Expression in Mammalian Cells, Elsevier, Amsterdam, 2003).

Fig. 4 zeigt schematisch die Menge an transient im Kulturüberstand exprimiertem hAAT (Balken) und die Lebendzellzahl (Linien) zu verschiedenen Zeitpunkten nach Transfektion eines Plasmids ohne SV40 ori (hAAT/Zellzahl - ori, Plasmid pGS 116) bzw. mit SV40 ori (hAAT/Zellzahl +ori, pGS151).

Fig. 5 zeigt schematisch die Menge an transient im Kulturüberstand exprimiertem hAAT (Balken) und die Lebendzellzahl (Linien) zu verschiedenen Zeitpunkten nach Transfektion von pGS151 (mit SV40 ori) in CAP-T und HEK293T Zellen.

Fig. 6 zeigt schematisch die intrazelluläre Kopienzahl der Plasmide pGS116 (ohne SV40 ori) bzw. pGS151 (mit SV40 ori) zu verschiedenen Zeitpunkten nach Transfektion in CAP-T und HEK293-T Zellen.

Fig. 7 zeigt schematisch die Menge an transient im Kulturüberstand exprimiertem hAAT (Balken) und die Lebendzellzahl (Linien) zu verschiedenen Zeitpunkten nach Transfektion von pGS151 (mit SV40 ori) in CAP-T mit Polyethylenimin (PEI) als Transfektionsreagenz.

Unter dem Begriff "Amniozyten" wie hier verwendet, versteht man im weiteren Sinne alle Zellen, die im Fruchtwasser vorhanden sind und durch Fruchtwasserpunktion gewonnen werden können. Die Zellen stammen entweder vom Amnion oder von fetalem Gewebe, das im Kontakt mit der Amnionflüssigkeit ist. Es wurden drei Hauptklassen von Amniozyten beschrieben, die auf Grund morphologischer Kriterien unterschieden werden: Fibroblastenartige Zellen (F-Zellen), epitheloide Zellen (E-Zellen) und Amnionflüssigkeitszellen (Amniotic Fluid Cells, AF-Zellen) (Hohn et al., Pediat. Res. 8:746-754, 1974). AF-Zellen sind der vorherrschende Zelltyp.

Unter dem Begriff "Expressionskassette" wird insbesondere ein Nukleinsäuremolekül bzw. ein Bereich eines Nukleinsäuremoleküls verstanden, das ein vor der kodierenden Region liegendes regulatorisches Element oder Promotor, eine kodierende Region bzw. einen offenen Leserahmen, sowie ein hinter der kodierenden Region liegendes transkriptionelles Terminationselement enthält. Das vor der kodierenden Region liegende regulatorische Element bzw. der Promotor kann ein konstitutiver, d.h. permanent die Transkription aktivierender Promotor (z.B. CMV-Promotor) oder ein regulierbarer, d.h. an- und/oder abschaltbarer Promotor (z.B. Tetrazyklin-regulierbarer Promotor) sein. Die kodierende Region der Expressionskassette kann ein durchgehender offener Leserahmen wie bei einer cDNA mit einem Startcodon am 5'-Ende und einem Stoppcodon am 3'-Ende sein. Die kodierende Region kann aus einer genomischen oder einer neu kombinierten Anordnung von kodierenden Exons und dazwischenliegenden, nicht-codierenden Introns bestehen. Die kodierende Region der Expressionskassette kann aber auch aus mehreren offenen Leserahmen bestehen, die durch sog. IRES (Internal Ribosome Entry Sites) voneinander getrennt sind.

Unter dem Begriff "permanente Zelllinien" wie hier verwendet, versteht man Zellen, die derart genetisch verändert sind, dass sie unter geeigneten Kulturbedingungen permanent in Zellkultur weiterwachsen können. Solche Zellen werden auch immortalisierte Zellen genannt.

Unter dem Begriff "Polypeptid" oder "rekombinantes Polypeptid" wie hier verwendet, werden Peptide aus mindestens 2 Aminosäuren verstanden. Das Polypeptid kann ko- und/oder posttranslational modifiziert werden, z.B. durch das Anhängen von Zuckerresten oder durch Modifikation von Aminosäureresten. Das Polypeptid kann linear, zirkulär oder verzweigt sein. Ferner kann das Polypeptid aus mehr als einer Aminosäurekette bestehen, wobei die Ketten durch intra- und/oder intermolekulare Bindungen mehr oder weniger komplexe Raumstrukturen einnehmen können (z.B. Sekundär-, Tertiär-, Quartärstruktur). Besteht das Polypeptid aus einer Aminosäurekette, kann diese auch durch intramolekulare Bindungen mehr oder weniger komplexe Raumstrukturen einnehmen. Die Polypeptide können pharmakologisch oder immunologisch aktive Polypeptide oder für diagnostische Zwecke verwendete Polypeptide sein.

Unter dem Begriff primäre Zellen" wie hier verwendet, versteht man Zellen, die durch direkte Entnahme aus einem Organismus oder einem Gewebe erhalten und in Kultur genommen wurden. Primäre Zellen besitzen nur eine sehr begrenzte Lebensdauer.

Unter dem Begriff "Produktionszelllinien" wie hier verwendet, versteht man permanente Zelllinien, die durch Einbringen eines Transgens, welches das zu produzierende gewünschte Polypeptid kodiert, stabil genetisch verändert wurden.

Unter dem Begriff "CAP" wie hier verwendet, versteht man permanente humane Amniozytenzelllinien, die durch Immortalisierung von primären humanen Amniozyten mit adenoviralen Genfunktionen E1A und E1B generiert worden sind.

Unter dem Begriff "CAP-T" wie hier verwendet, versteht man CAP-Zellen, die zusätzlich mit einem Nukleinsäuremolekül enthaltend die Sequenz des SV40 großen T-Antigens stabil transfiziert wurden.

Unter dem Begriff "Transfektion" wie hier verwendet, wird jedes Verfahren verstanden, das sich zum Einbringen der genannten Nukleinsäure(n) in die Zellen eignet. Als Beispiele sind die klassische Kalziumphosphat-Methode, Elektroporation, liposomale Systeme jeglicher Art und Kombinationen dieser Verfahren zu nennen.

Unter dem Begriff "transiente Expression" wie hier verwendet, versteht man jedes Verfahren, bei dem durch Transfektion Nukleinsäure(n) in die Zelle eingebracht werden, ohne dass durch ein geeignetes Selektionsverfahren stabile Zelllinien selektioniert werden, die auf Dauer in der Zellkultur weiter kultiviert werden können.

Unter dem Begriff "stabile Expression" wie hier verwendet versteht man die Expression eines Transgens in Produktionszelllinien.

Unter dem Begriff "Transgen" wie hier verwendet versteht man die ein rekombinantes Polypeptid kodierende Nukleinsäuresequenz.

Ein Gegenstand der vorliegenden Erfindung betrifft ein Verfahren zur Herstellung einer permanenten humanen Amniozyten-Zelllinie, das die folgenden Schritte umfasst:
a) Transfizieren primärer humaner Amniozyten-Zellen mit einem Nukleinsäuremolekül umfassend eine Nukleinsäuresequenz kodierend für die adenoviralen Genfunktionen E1A und, E1B; sog. 1. Transfektion und
b) anschließende Transfektion der permanenten humanen Amniozyten-Zellinie mit einem Nukleinsäuremolekül umfassend eine Nukleinsäuresequenz kodierend für das große T-Antigen von SV40, sog. 2. Transfektion.

Vorzugsweise umfasst das Nukleinsäuremolekül aus Schritt b) des erfindungsgemäßen Verfahrens zur Herstellung einer permanenten humanen Amniozyten-Zelllinie eine Nukleinsäuresequenz kodierend für eine nicht-sezernierte Form des großen T-Antigens von SV40.

Bei der Transfektion in Schritt b) des erfindungsgemäßen Verfahrens wird die permanente humane Amniozyten-Zelllinie alternativ mit einem Nukleinsäuremolekül transfiziert, umfassend eine Nukleinsäuresequenz kodierend für das Epstein-Barr-Virus (EBV) Nuclear Antigen-1 (EBNA-1), sog. 2. Transfektion. Vorzugsweise umfasst das Nukleinsäuremolekül eine Nukleinsäuresequenz kodierend für eine nicht-sezernierte Form des Epstein-Barr-Virus (EBV) Nuclear Antigen-1 (EBNA-1).

Durch die im erfindungsgemäßen Verfahren durchgeführten Transfektionen werden die primären humanen Amniozyten-Zellen bevorzugt stabil transfiziert, d.h. die transfizierte DNA wird in das Genom der Zelle integriert.

Durch die Transfektion der primären humanen Amniozyten-Zellen mit dem Nukleinsäuremolekül umfassend die für E1A- und E1B-kodierenden Nukleinsäuresequenzen, werden die Zellen immortalisiert. Das zur Immortalisierung der primären humanen Amniozyten-Zellen verwendete Nukleinsäuremolekül umfasst E1A- und E1B-Nukleinsäuresequenzen, die vorzugsweise von humanen Adenoviren stammen, insbesondere vom humanen Adenovirus Serotyp 5. In einer bevorzugten Ausführungsform umfasst das zur Immortalisierung verwendete Nukleinsäuremolekül, neben den E1A und E1B-kodierenden Nukleinsäuresequenzen, die Nukleinsäuresequenz kodierend für die adenovirale pIX-Genfunktion. Das pIX-Polypeptid, ein virales Strukturprotein, wirkt als Transkriptionsaktivator auf verschiedene virale und zelluläre Promotoren wie z.B. den Thymidinkinase- und den Beta-Globin-Promotor.

Eine beispielhafte Sequenz findet sich in Genbank Acc. Nr. X02996. Insbesondere umfassen die Nukleinsäuremoleküle die Nukleotide 1 bis 4344 (SEQ ID NO:1 umfasst Nukleinsäuresequenzen kodierende für E1A, E1B und pIX), 505 bis 3522 (SEQ ID NO:2 umfasst Nukleinsäuresequenzen kodierend für E1A und E1B) oder die Nukleotide 505 bis 4079 (SEQ ID NO:3 umfasst Nukleinsäuresequenzen kodierend E1A, E1B und pIX) des humanen Adenovirus Serotyp 5.

Insbesondere werden die humanen Amniozyten-Zellen mit den zu exprimierenden Nukleinsäuresequenzen kodierend für die gewünschte Genfunktion in Form einer Expressionskassette transfiziert. Die Expressionskassette umfasst ein Nukleinsäuremolekül, die ein vor der kodierenden Region liegendes regulatorisches Element oder Promotor, eine kodierende Region bzw. einen offenen Leserahmen, sowie ein hinter der kodierenden Region liegendes transkriptionelles Terminationselement enthält.

Die Expressionskassette oder das Nukleinsäuremolekül enthält in einer Ausführungsform insbesondere die Nukleinsäuresequenz für das große SV40 T-Antigen (SEQ ID NO:4), die Nukleinsäuresequenz für einen Promotor ausgewählt aus der Gruppe CMV-Promotor (SEQ ID NO:5), CAG-Promotor (Niwa et al., Gene 108:193-199, 1991) und RSV-Promotor (Genbank Acc. Nr.DQ075935), die Sequenz für SV40 SD/SA (intron) (SEQ ID NO:6) und die Nukleinsäuresequenz für SV40 polyA (SEQ ID NO:7).

Die Expressionskassette oder das Nukleinsäuremolekül enthält in einer weiteren Ausführungsform insbesondere die Nukleinsäuresequenz für das Epstein-Barr-Virus (EBV) Nuclear Antigen-1 (EBNA-1) (SEQ ID NO:8), die Nukleinsäuresequenz für einen Promotor ausgewählt aus der Gruppe CMV-Promotor (SEQ ID NO:5), CAG-Promotor (Niwa et al., Gene 108:193-199, 1991) und RSV-Promotor (Genbank Acc. Nr.DQ075935), die Nukleinsäuresequenz für SV40 SD/SA (intron) (SEQ ID NO:6) und die Nukleinsäuresequenz für SV40 polyA (SEQ ID NO:7).

Die primären humanen Amniozyten-Zellen werden durch direkte Entnahme aus dem Organismus oder einem dem Organismus entnommenen Gewebe erhalten und in Kultur genommen.

Das erfindungsgemäße Verfahren kann auch anstelle des Schritts a) mit bereits vorhandenen immortalisierten humanen Amniozyten-Zelllinien durchgeführt werden, die in ihrem Genom die Nukleinsäuresequenzen für die adenoviralen Genfunktionen E1A und E1 B aufweisen. Vorzugsweise umfassen die immortalisierten humanen Amniozyten-Zelllinien in ihrem Genom die Nukleinsäuresequenzen für die adenoviralen Genfunktionen E1A, E1B und pIX. Die vorhandenen immortalisierten humanen Amniozyten-Zelllinien werden bei Bedarf mit den vorstehend beschriebenen Nukleinsäuremolekül enthaltend eine Expressionskassette kodierend für das große T-Antigen von SV40 oder das Epstein-Barr-Virus (EBV) Nuclear Antigen-1 (EBNA-1) transfiziert. Der Fachmann erkennt, dass die 2. Transfektion zeitlich nur insoweit abhängig von der 1. Transfektion der primären humanen Zelle ist, als dass sie nach der 1. Transfektion erfolgen muss. Es ist nicht erforderlich, dass die 2. Transfektion unmittelbar nach der 1. Transfektion erfolgen muss. So können auch bereits seit etlichen Jahren etablierte immortalisierte humane Amniozyten-Zelllinien, die mit DA und/oder E1 B immortalisiert worden sind, bei Bedarf mit den vorstehendem Nukleinsäuremolekül in einer 2. Transfektion transfiziert werden.

Die vorliegenden Erfindung beschreibt eine mit dem erfindungsgemäßen Verfahren hergestellte permanente humane Amniozyten-Zelllinie, umfassend die Nukleinsäuresequenzen für die adenoviralen Genfunktionen EIA und E1B und die Nukleinsäuresequenz für das große T-Antigen von SV40 oder das Epstein-Barr-Virus (EBV) Nuclear Antigen-1 (EBNA-1). Beschrieben wird ferner eine permanente humane Amniozyten-Zelllinie, umfassend die Nukleinsäuresequenzen für die adenoviralen Genfunktionen E1A, E1B und pIX und die Nukleinsäuresequenz für das große T-Antigen von SV40 oder das Epstein-Barr-Virus (EBV) Nuclear Antigen-1 (EBNA-1).

Die vorliegende Erfindung beschreibt ein Verfahren zur transienten Expression von rekombinanten Polypeptiden oder Proteinen unter Verwendung der mit dem erfindungsgemäßen Verfahren hergestellten permanenten humanen Amniozyten-Zelllinie umfassend die folgenden Schritte:
a) Transfizieren der permanenten humanen Amniozyten-Zellline mit einem Nukleinsäuremolekül umfassend eine Nukleinsäuresequenz kodierend für ein gewünschtes rekombinantes Polypeptid oder Protein und eine Erkennungs- oder Bindungsstelle für das große T-Antigen von SV40 oder das Epstein-Barr-Virus (EBV) Nuclear Antigen-1 (EBNA-1),
b) Kultivieren der in Schritt a) erhaltenen transfizierten permanenten humanen Amniozyten-Zelllinie unter Bedingungen, die die Expression des gewünschten rekombinanten Polypeptids oder Proteins erlauben, und anschließend
c) Isolieren des gewünschten rekombinanten Polypeptids oder Proteins aus den Zellen oder aus dem Kulturüberstand.

Enthält die erfindungsgemäß hergestellte permanente humane Amniozyten-Zelllinie ein Nukleinsäuremolekül umfassend die Nukleinsäuresequenz kodierend für das große T-Antigen von SV40, wird die Zelllinie z.B. mit einem Expressionsplasmid transfiziert, das eine Expressionskassette oder ein Nukleinsäuremolekül umfassend eine Nukleinsäuresequenz kodierend für das zu exprimierende Transgen und den SV40-Replikationsursprung (SV40 ori) enthält. Das in der Zelllinie stabil intrazellulär exprimierte große T-Antigen von SV40 bindet an den SV40-Replikationsursprung des durch Transfektion in die Zelllinie eingebrachten Expressionsplasmids und bewirkt eine episomale Replikation des Expressionsplasmids und dadurch eine Amplifizierung der Kopienzahl des zu exprimierenden Transgens. Das vom Transgen kodierte gewünschte Genprodukt kann nach Kultivierung der Zellen für einige Tage aus den Zellen oder aus dem Kulturüberstand gewonnen werden. Das Transgens wird somit transient exprimiert.

Enthält die erfindungsgemäß hergestellte permanente humane Zelllinie ein Nukleinsäuremolekül umfassend die Nukleinsäuresequenz kodierend für das Epstein-Barr- Virus (EBV) Nuclear Antigen-1 (EBNA-1), wird die Zelllinie z.B. mit einem Expressionsplasmid transfiziert, das eine Expressionskassette oder ein Nukleinsäuremolekül umfassend eine Nukleinsäuresequenz kodierend für das zu exprimierende Transgen und den EBV-Replikationsursprung (EBV oriP) (Durocher et al., Nucleic Acids Research Vol. 30 Nr. 2 e9, 2002; Tuvesson et al. Cytotechnology 56:123-136, 2008) enthält. Das in der Zelllinie stabil intrazellulär exprimierte EBNA-1 von EBV bindet an den oriP-Replikationsursprung des durch Transfektion in die Zelllinie eingebrachten Expressionsplasmids und bewirkt eine episomale Replikation des Expressionsplasmids und dadurch eine Amplifizierung der Kopienzahl des zu exprimierenden Transgens. Das vom Transgen kodierte gewünschte Genprodukt kann nach Kultivierung der Zellen für einige Tage aus den Zellen oder aus dem Kulturüberstand gewonnen werden. Das Transgens wird somit transient exprimiert. Die erfindungsgemäß hergestellten Zellen können unter den üblichen Bedingungen für die Kultivierung von eukaryotischen Zellen bei etwa 37°C, 95% Luftfeuchtigkeit und 8% CO₂ kultiviert werden. Die erfindungsgemäß hergestellten Zellen können in serum-haltigem oder serum-freiem Medium, in adhaerenter Kultur oder in Suspensionskultur kultiviert werden. Die Kultivierung in Suspension kann in verschiedensten Fermentationsgefäßen erfolgen, z.B. in Rührkesselreaktoren, "Wave"-Reaktoren, in Shaker- oder Spinnergefäßen oder in sog. "Roller Bottles". Die Zellen sind somit für eine Prozess-Expandierung im industriellen Maßstab geeignet. Die Transfektion der Zellen zur transienten Expression kann mit verschiedensten Transfektionsmethoden erfolgen, die oben beschrieben wurden. Transfektion und transiente Expression können auch im "High-Throughput"-Format bzw. -Screening durchgeführt werden, z.B. im 96- oder 384-Well-Format.

T-Antigen von Simian Virus 40 (SV40) ist ein multifunktionales Phosphoprotein, das sowohl die virale Replikation als auch die zellulären Funktionen nach Infektion kontrolliert. T-Antigen ist ein transformierendes Agens und greift über Interaktion mit dem Tumorsupressorprotein p53 in den Zellzyklus ein. Während der Replikation des viralen Genoms wird T-Antigen als DNA-Helikase benötigt, um das doppelsträngige Genom zu entwinden. T-Antigen ist das einzige virale Protein, das für die Replikation benötigt wird. Die anderen Funktionen werden von zellulären Proteinen erfüllt. Im ersten Schritt der DNA-Replikation binden 12 T-Antigen-Moleküle als Doppelhexamere an den Startpunkt der DNA-Replikation (ori) im SV40 Genom. An diesen Helikase-Komplex binden dann die nötigen zellulären Proteine wie DNA-Polymerase und entwinden und replizieren die DNA. Der sog. "minimale ori" besteht aus einer 63-bp-langen "Core"-Sequenz. Bei einer transienten Transfektion von zirkulären Plasmiden in die Zielzelle erfolgt keine Integration in das Wirtsgenom, was dazu führt, dass die Plasmidkonzentration nach Zellteilung stetig abnimmt und die Expression eines auf dem Plasmid liegenden kodierenden Gens nur vorübergehend ist. Das Einbringen des SV40 ori-Fragments in das Expressionsplasmid und die Expression des SV40 T-Antigens in der Produktionszelle führen zu einer erhöhten Kopienzahl des Plasmids und somit zu einer erhöhten Expressionsleistung.

Das erfindungsgemäße Verfahren zur transienten Expression von Polypeptiden und Proteinen hat den Vorteil, dass es hinsichtlich der Quantität und Qualität des rekombinanten Genproduktes leistungsfähiger und damit im Gesamtprozess der industriellen Entwicklung von Protein-basierten Therapeutika auch kostengünstiger ist als bisher verwendete Verfahren. Insbesondere ist von Vorteil, dass ein hocheffizientes transientes Expressionssystem auf der Grundlage einer humanen Zelllinie bereitgestellt wird, die erstens im Gegensatz zu nicht-humanen Säugerzellen oder Nicht-Säugerzellen humane Proteine authentisch posttranslational modifiziert, und zweitens gleichermaßen gut für die Etablierung stabiler Produktionszelllinien im industriellen Herstellungsprozess geeignet ist. Auf diese Weise kann sichergestellt werden, dass im Zuge der Entwicklung diagnostischer oder therapeutischer Produkte qualitative Merkmale des Genproduktes nach transienter Expression in der frühen Entwicklungsphase und nach stabiler Expression in permanenten Produktionszelllinien in der späten Phase und industriellen Herstellung größtmögliche Identität insbesondere im Hinblick auf Unterschiede in diesen Merkmalen, die in der Natur des Zellsystems begründet sein können, aufweisen.

Ein weiterer Vorteil der vorliegenden Erfindung besteht darin, dass die erfindungsgemäß hergestellten permanenten humanen Zelllinien hohe Expressionsausbeuten bei transienter Expression aufweisen. So wurden bei transienter Expression in SV40-T-Antigenproduzierenden Amniozyten-Zelllinien nach Transfektion mit einem Plasmidvektor, der außer der kodierenden Sequenz für das gewünschte Genprodukt einen SV40-Replikationsursprung (SV40 ori) trägt, unerwarteterweise sehr hohe Produktausbeuten im Kulturüberstand von bis zu 60 mg/Liter gefunden. Die Produktausbeuten waren mehr als 70-fach höher als bei transienter Expression in einer Amniozyten-Zelllinie, die kein T-Antigen exprimiert.

Ein weiterer Vorteil der erfindungsgemäß hergestellten permanenten humanen Zelllinie besteht darin, dass ein humanes Zellsystem basierend auf immortalisierten humanen Amniozyten bereitgestellt wird, das sich sowohl für die transiente Expression von Proteinen als auch für die stabile Expression von Proteinen in permanenten Produktionszelllinien (Schiedner et al., BMC Biotechnology 8, 13, 2008) eignet. Gegenüber der Verwendung unterschiedlicher Zellsysteme für die transiente Expression (z.B. HEK293 oder HEK293-Varianten) und die stabile Expression (z.B. CHO) wird somit das Risiko minimiert, dass sich strukturelle oder funktionelle Eigenschaften der Expressionsprodukte aus transienter und stabiler Produktion, die in der Natur des Expressionssystems begründet liegen, unterscheiden. Dadurch wird der Entwicklungsprozess besser planbar, weniger zeitaufwendig und kostengünstiger.

Die Nukleinsäuresequenzen für die Expression des mindestens einen rekombinanten Polypeptids liegen in mindestens einer Expressionskassette vor. Die Expressionskassetten enthalten Promotoren und transkriptionelle Terminationssequenzen. Als Promotoren können z.B. CMV- (Cytomegalievirus-) Promotor (Makrides, 9-26 in: Makrides (Hrsg.), Gene Transfer and Expression in Mammalian Cells, Elsevier, Amsterdam, 2003), EF-1α-Promotor (Kim et al., Gene 91:217-223, 1990), CAG-Promotor (ein Hybridpromotor aus dem Immediate Early-Enhancer des humanen Cytomegalievirus und einem modifizierten Huhn β-Aktin Promotor mit erstem Intron) (Niwa et al., Gene 108:193-199, 1991), humaner oder muriner pgk- (Phosphoglyceratkinase-) Promotor (Adra et al., Gene 60:65-74, 1987), RSV-(Rous Sarkoma Virus-) Promotor (Makrides, 9-26 in: Makrides (Hrsg.), Gene Transfer and Expression in Mammalian Cells, Elsevier, Amsterdam, 2003) oder SV40- (Simian Virus 40-) Promotor (Makrides, 9-26 in: Makrides (Hrsg.), Gene Transfer and Expression in Mammalian Cells, Elsevier, Amsterdam, 2003) dienen. Als Polyadenylierungsstellen können z.B. die Polyadenylierungssequenzen des SV40 Large T-Antigens (Genbank Acc. Nr. J02400) oder des humanen G-CSF- (Granulozyten-Kolonie-stimulierender Faktor, granulocyte colony stimulating factor) Gens (Mizushima und Nagata, Nucl. Acids Res. 18:5322, 1990) dienen.

Die vorliegende Erfindung beschreibt das Polypeptid oder Protein, das unter Verwendung des erfindungsgemäßen Verfahrens erhalten wird.

Das rekombinante Polypetid des erfindungsgemäßen Verfahrens kann ein therapeutisches Protein wie z.B. humanes Alpha 1-Antitrypsin oder Wachstumsfaktoren wie Erythropoietin oder Interleukin-2 sein. Humanes Alpha 1-Antitrypsin (hAAT) ist ein Proteinase-Inhibitor, der Elastase und andere Proteinasen inhibiert und bei erblichem hAAT-Mangel, der zu schweren Lungen- und Leberschädigungen führt, therapeutisch wirksam ist. Bei Erythropoietin handelt es sich um einen wichtigen Wachstumsfaktor für Erythrozyten (rote Blutkörperchen), der bei Anämie sowie bei Transplantationspatienten blutbildende Wirkung hat. Interleukin-2 (Il-2) zählt zu den zellulären Botenstoffen des Immunsystems und hat große Bedeutung bei der Aktivierung der zellulären Immunantwort beispielsweise bei Tumorerkrankungen. Zu den therapeutisch wirksamen Polypeptiden zählen auch Blutgerinnungsfaktoren wie z.B. Faktor VIII und Faktor IX, die bei Hämophilie-Patienten mit Gerinnungsstörungen eingesetzt werden. Das rekombinante Polypeptid des erfindungsgemäßen Verfahrens kann ein Hormon sein. Biotechnologisch hergestellte Hormone werden in der Substitutionstherapie bei Patienten mit hormonellen Störungen verwendet. Beispiele sind das Blutzuckersenkende Hormon Insulin, auf das viele Diabetes mellitus-Patienten angewiesen sind, Somatotropin (Wachstumshormon) für die Behandlung von Zwergwüchsigkeit, und gonadotrope Faktoren wie Follikelstimulierendes Hormon (FSH) oder Luteinisierendes Hormon (LH) für die Behandlung von Fertilitätsstörungen. Das rekombinante Polypeptid kann weiterhin ein Enzym sein, das intrazellulär oder im Kulturüberstand gleichzeitig exprimierte andere rekombinante Polypeptide posttranslational modifiziert, z.B. an der Glykosylierung beteiligtes Enzym. Die in der erfindungsgemäßen permanenten humanen Zelllinie exprimierten Genprodukte E1A, E1B und pIX, sowie das große T-Antigen von SV40 und das Epstein-Barr-Virus (EBV) Nuclear Antigen-1 (EBNA-1) zählen nicht zu dem zu produzierenden gewünschten Polypeptid.

Das rekombinante Polypetid des erfindungsgemäßen Verfahrens kann ein rekombinanter Antikörper sein, der zu therapeutischen oder diagnostischen Zwecken eingesetzt werden kann. Antikörper gegen den Tumor-Nekrose-Faktor alpha (TNF-α) werden bei Patienten mit rheumatoider Arthritis, Antikörper gegen den zellulären Rezeptor des Epidermalen Wachstumsfaktors (EGFR) bei Krebspatienten eingesetzt. Für diagnostische Zwecke eingesetzte Antikörper können beispielsweise Bestandteile von kommerziellen Diagnose-Kits sein, die auf Verfahren wie Enzym-gekoppelte Immun-Adsorptions-Tests (Enzyme-linked Immuno Sorbent Assay, ELISA) oder radioaktiven Immun-Adsorptions-Tests (Radio Immuno Sorbent Assay, RIA) beruhen. Die Antikörper dienen in diesen Testverfahren zum Nachweis der Antigene von Infektionserregern, wie beispielsweise dem humanen Hepatitis B-Virus.

Antikörper oder Immunglobuline (Ig) setzen sich aus einer schweren und einer leichten Kette zusammen, die jeweils aus variablen und konstanten Regionen oder Domänen bestehen. Die Nukleinsäuresequenzen der transfizierten Nukleinsäuremoleküle zur Expression eines Antikörpers können zwei getrennte Expressionskassetten enthalten, von denen die eine die leichte Kette, die andere die schwere Kette des Immunglobulinmoleküls kodiert. Nach Expression beider Ketten in der erfindungsgemäßen Zelle lagern sich diese zum aktiven Antikörpermolekül zusammen. Die Expressionskassetten der beiden Ketten können dabei auf getrennten oder auf demselben Nukleinsäuremolekül liegen. Die kodierenden Sequenzen für die leichte und die schwere Kette können aber auch innerhalb derselben Expressionskassette liegen und durch eine IRES-Sequenz (internal ribosome entry site, interne Ribosomenbindungsstelle) getrennt werden, die eine Expression sowohl der schweren als auch der leichten Kette gewährleistet. Die kodierenden Sequenzen für die leichte und die schwere Kette können prinzipiell auch innerhalb derselben Expressionskassette liegen und durch eine Sequenz getrennt werden, die eine enzymatische Spaltstelle für eine Proteinase (z.B. Thrombin) kodiert, welche dann in der Zelle gleichzeitig exprimierte wird und das Vorläufer-Polypeptid aus Leicht- und Schwerkettensequenz in aktive leichte und schwere Kette spaltet.

Rekombinante Antikörper, die von der Nukleinsäuresequenz in der erfindungsgemäß hergestellten Zelle kodiert werden, können auch aus Fragmenten eines Antikörpers bestehen anstelle der kompletten leichten und schweren Kette. Sogenannte Einzelketten-Antikörper (scFv, single chain variable fragments) bestehen aus den variable Domänen einer schweren und einer leichten Kette, die durch eine Aminosäuresequenz (einen sog. Linker) verbunden werden, die eine freie Beweglichkeit beider Domänen gewährleistet. Durch intramolekulare Zusammenlagerung beider Domänen entsteht eine antigenbindende Struktur, die dem variablen Bereich eines Immunglobulinmoleküls entspricht. Bispezifische Einzelketten-Antikörper (bis-scFv) bestehen aus zwei solchen Einzelketten-Anordnungen aus den variablen Domänen einer schweren und einer leichten Kette, die ihrerseits wiederum durch eine verbindende Sequenz zusammenhängen und gegeneinander beweglich sind; solche Moleküle können an zwei antigene Bindungsstellen (Epitope) gleichzeitig binden und dadurch zwei molekulare Strukturen nicht-kovalent verbinden. Bispezifische Diabodies bestehen aus zwei getrennt exprimierten Einzelketten, die jeweils variable Domänen einer leichten und einer schweren Kette, getrennt nur durch einen sehr kurzen oder ganz ohne Linker, enthalten. Der kurze oder fehlende Linker verhindert die intramolekulare Zusammenlagerung, durch intermolekulare Zusammenlagerung einer variablen schweren und leichten Domäne entsteht wiederum ein aktives Molekül mit zwei Bindungsvalenzen.

Das von dem im vorliegenden Verfahren transfizierte Nukleinsäuremolekül kodierte rekombinante Polypetid kann ein virales, bakterielles oder parasitäres Protein sein, das für eine Verwendung als prophylaktische oder therapeutische Impfstoffe (Vakzine) produziert werden soll. Dabei kann es sich sowohl um ein Strukturpolypeptid als auch um ein regulatorisch oder enzymatisch aktives Polypeptid von Viren, Bakterien oder Parasiten handeln. Ein virales Protein kann z.B. das Hepatitis B-Virus Oberflächen-Antigen (HBV Surface Antigen) oder das Strukturprotein L1 von humanen Papillomviren sein. Ein bakterielles Protein, das nach Expression in Produktionszelllinien für die Impfstoffherstellung in Frage kommt, sind z.B. Enterotoxin-Untereinheiten von enterotoxinogenen Escherichia coli (ETEC) oder Transferrin-bindende Proteine (Transferrin binding proteins, Tbp A und B) von Neisseria gonorrhoeae. Ein Polypeptid von Parasiten, das von dem im vorliegenden Verfahren transfizierte Nukleinsäuremolekül kodiert werden könnte, ist z.B. das Merozoiten-Oberflächenprotein (Merozoite Surface Protein, MSP) des Malariaerregers Plasmodium falciparum oder Glutathion S-transferase (GST) von Schistosoma japonicum.

Das von dem im vorliegenden Verfahren transfizierte Nukleinsäuremolekül kodierte rekombinante Polypetid kann außerdem ein virales Protein sein, das in Zelllinien eine Produktion von rekombinanten viralen Gentransfer-Vektoren erlaubt. Dieses virale auch Komplementationsfaktor genannte Protein wird in der Zelllinie exprimiert und ist die für die Produktion der Gentransfer-Vektoren notwendige enzymatische oder strukturelle Komponente, die nicht auf dem Nukleinsäuremolekül des Gentransfer-Vektors kodiert wird. In solchen Gentransfer-Vektoren sind in der Regel aus Sicherheitsgründen bestimmte virale Genfunktionen deletiert. Zu den Gentransfer-Vektoren, deren Komplementationsfaktoren durch mit dem beschriebenen Verfahren eingebrachte Transgene kodiert werden können, gehören beispielsweise Vektoren, die auf Adenovirus, Adenovirus-Assoziiertern Virus (AAV), Retro- oder Lentivirus, oder Herpesvirus basieren. Der in der Zelllinie exprimierte Komplementationsfaktor kann auch deletierte oder rekombinante Viren bei ihrer Produktion komplementieren, die kein zu transferierendes Gen enthalten und damit nicht als Gentransfer-Vektoren fungieren, sondern z.B. als Impfstoff verwendet werden.

Das mit dem vorliegenden Verfahren transient exprimierte Polypeptid kann ferner ein Rezeptorpolypeptid sein, das insbesondere auf der Oberfläche der Zelle lokalisiert ist und für die Infektion der Zelle durch ein Virus bzw. die Transduktion der Zelle durch einen viralen Gentransfer-Vektor verantwortlich ist. Als viraler Rezeptor für den initialen Schritt der Infektion von Zellen mit dem Adenovirus Serotyp 2 oder 5, von denen die meisten konventionellen adenoviralen Vektoren abgeleitet sind, wurde der sog. Coxsackie- und Adenovirus-Rezeptor, CAR, identifiziert (Bergelson et al., Science 275:1320-1323, 1997).

Die hinreichende Expression von CAR auf der Oberfläche ist eine Voraussetzung dafür, dass sich eine Zelle als Produktionszelle für adenovirale Gentransfer-Vektoren eignet. In einer bevorzugten Ausführungsform ist das rekombinante Polypeptid der Coxsackie- und Adenovirus-Rezeptor (CAR). Die Überexpression des Rezeptorpolypeptids kann die Infizierbarkeit und damit die Produktionseffizienz dieser Zellen hinsichtlich adenoviraler Vektoren deutlich verbessern. Ferner kann das Nukleinsäuremolekül außer CAR sekundäre Rezeptoren oder Internalisierungsrezeptoren kodieren, wie z.B. bestimmte Integrine, die die Aufnahme des Virus bzw. Gentransfer-Vektors in die Zelle vermitteln und deren zusätzliche Expression bei der Herstellung von Produktionszellen für adenovirale Vektoren sinnvoll sind.

Das beschriebene Verfahren kann unter anderem zur Produktion therapeutischer Polypeptide, Blutgerinnungs- und Wachstumsfaktoren, Hormone und Antikörper sowie viraler, bakterieller oder parasitärer Polypeptide zur Verwendung als Impfstoff verwendet werden. Ferner sind die erfindungsgemäß hergestellten Zellen zur Produktion von diagnostisch relevanten Proteinen verwendbar, wie z.B. virale, bakterielle oder parasitäre Antigene oder entsprechende spezifische Antikörper. Ferner sind die erfindungsgemäß hergestellten Zellen zur Produktion von technisch oder industriell relevanten Proteinen verwendbar, z.B. von Enzymen zur Katalyse technischer Syntheseprozess oder zum Abbau von Schadstoffen. Die erfindungsgemäß hergestellten Zellen können ein oder auch mehrere verschiedene rekombinante Polypeptide exprimieren. Die Anzahl der exprimerbaren Polypeptide hängt davon ab, wie viele verschiedene Nukleinsäuresequenzen kodierend für die rekombinanten Polypeptide mit dem erfindungsgemäßen Verfahren in die Zellen transient transfiziert werden.

Die vorliegende Erfindung betrifft ferner die Verwendung der mit dem erfindungsgemäßen Verfahren hergestellten permanenten humanen Amniozyten-Zelllinien zur Herstellung eines Polypeptids oder Proteins.

Die folgenden Beispiele erläutern die Erfindung und sind nicht als einschränkend aufzufassen. Sofern nicht anders angegeben, wurden molekularbiologische Standardmethoden verwendet, wie z.B. von Sambrook et al., 1989, Molecular cloning: A Laboratory Manual 2. Auflage, Cold Spring Harbor Laboratory Press, Cold Spring Harbor, New York, beschrieben.

### 1. Klonierungen

### a. Plasmide zur Transformation primärer Amniozyten: pSTK146, pGS119, pGS122

Plasmid pSTK146 wurde detailliert in EP 1 230 354 B1 beschrieben und umfasst den murinen Phösphoglyceratkinase (pgk) Promotor, Adenovirus Serotyp 5 (Ad5)-Sequenzen Nukleotid (nt.) 505 bis 3522 und das Spleiß- und Polyadenylierungssignal von SV40. Die adenoviralen Sequenzen in pSTK146 umfassen die kodierende E1A und E1B-Region, wobei die Expression von E1A vom pgk Promoter aus reguliert wird.

Plasmid pGS 119 wurde ausführlich in WO 2007/056994 beschrieben und enthält den murinen pgk Promoter, Ad5 Sequenzen nt. 505-3522 (umfassend die E1A und E1B-Region), das Spleiß- und Polyadenlierungssignal von SV40, gefolgt von der pIX-Region von Ad5 nt. 3485-4079.

Plasmid pGS122 wurde ausführlich in WO 2007/056994 beschrieben und enthält die adenoviralen Sequenzen nt. 1-4344, umfassend die E1A, E1B und pIX Regionen inklusive der jeweiligen regulatorischen Promoter- und Polyadenylierungssequenzen. Die adenoviralen Sequenzen in pGS122 werden flankiert von PmeI-Schnittstellen.

### b. T-Antigen-Expressionsplasmide: pGS158, pGS159, pGS161

Plasmide pGS158, pGS159 und pGS161 enthalten alle die Expressionskassette für T-Antigen von SV40 (SEQ ID NO:4), flankiert von einem Intron aus SV40 (SEQ ID NO:6) und einer Polyadenylierungsstelle (SEQ ID NO:7). Zusätzlich enthält pGS 158 den CAG-Promotor (Hybridpromotor bestehend aus dem CMV-Enhancer und dem Huhn β-Aktin-Promotor) (Niwa et al., Gene 108:193-199, 1991), pGS159 den RSV-Promotor (Promotor aus Rous Sarcomavirus) (Genbank Acc. Nr.DQ075935) und pGS161 den CMV-Promotor (früher Promotor des humanen Cytomegalie-Virus) (SEQ ID NO:5). Zur Generierung stabiler Zelllinien enthalten Plasmide pGS158, pGS159 und pGS161 eine Blasticidin-Expressionskassette mit dem Ubiquitinpromotor (pUB/Bsd, Invitrogen #V512-20). In einem ersten Schritt wurde ein 2,6 kb-Fragment, das die T-Antigen-kodierenden Sequenzen enthält, in das Plasmid pGS140 eingefügt. Das Plasmid pGS140 enthält den humanen CMV-Promotor (SEQ ID NO:5), eine Intron-Region aus SV40 mit Spleißdonor/Spleißakzeptorstelle (SEQ ID NO:6), eine singuläre NotI-Restriktionsschnittstelle und eine PolyA-Sequenz aus SV40 (SEQ ID NO:7). Zum Einfügen des T-Antigen-Fragments wurde pGS140 mit NotI linearisiert, der 5'-Überhang aufgefüllt und mit dem isolierten Fragment ligiert. Das so entstandene Plasmid erhielt die Bezeichnung pGS149.

Für Plasmid pGS 158 wurde pGS 149 mit XbaI verdaut und ein ca. 3-kb-Fragment, enthaltend die Intronsequenz, T-Antigen und PolyA-Sequenz, isoliert. Dieses Fragment wurde in die NotI-Schnittstelle (5'-Überhang aufgefüllt) von pGS 152 einfügt. pGS152 entstand durch Einfügen eines 1.1 kb großen CAG-Promotor-Fragments (Niwa et al., Gene 108:193-199, 1991) in die EcoRV-Schnittstelle von pUB/Bsd.

Für Plasmid pGS159 wurde das T-Antigen-enthaltende 3-kb-große XbaI-Fragment aus pGS149 in die aufgefüllte NotI-Schnittstelle von pGS153 eingefügt. pGS153 enthält ein ca. 0,6 kb großes RSV-Promotor-Fragment, eingefügt in die EcoRV-Schnittstelle von pUBBsd. Für Plasmid pGS161 wurde pGS149 mit SphI verdaut, die 3'-Überhänge aufgefüllt, das 3.6-kb-Fragment, enthaltend den CMV-Promotor, das SV40-Intron, die T-Antigen-Sequenz und das PolyA, isoliert und in die EcoRV-Schnittstelle von pUB/Bsd eingefügt.

### c. hAAT-Expressionsplasmide: pGS116, pGS151

Plasmid pGS116 wurde detailliert in EP1948789 beschrieben und enthält den humanen CMV-Promotor gefolgt von einer SV40-Spleißdonor/Spleißakzeptorstelle, der hAAT-cDNA (SEQ ID NO:12) und der SV40-Polyadenylierungsstelle.

Das Plasmid pGS151 (Abb. 2b) enthält diese hAAT-Expressionskassette und den Startpunkt der DNA-Replikation (*origin of replication,* ori) aus SV40. Mit Hilfe von SV40-DNA und der Primer ori 1 (CCGGAATTCTTTGCAAAAGCCTAGGCCTC)(SEQ ID NO:9) und ori 2 (CCGGAATTCTGAGGCGGAAAGAACCAGCT)(SEQ ID NO:10) wurden die SV40-Sequenzen durch Polymerasekettenreaktion (PCR) amplifiziert, mit EcoRI verdaut (je eine EcoRI-Schnittstelle lokalisiert in den Primern) und in die EcoRI-Schnittstelle von pGS116 eingefügt.

### d. Epo-Expressionsplasmide: pGS177

Plasmid pGS127 wurde detailliert in EP1948789 beschrieben und enthält den humanen CMV-Promotor gefolgt von einer SV40-Spleißdonor/Spleißakzeptorstelle, der cDNA für das humane Erythropoietin (Epo) und der SV40-Polyadenylierungsstelle.

Für Plasmid pGS177 wurde das ori-Fragment aus SV40 wie oben beschrieben mit den Primern ori 1 und ori 2 amplifiziert und in pGS127 eingefügt.

### 2. Überprüfung der Konstrukte

### a. Sequenzanalyse

Die Vollständigkeit aller oben beschriebenen Plasmide wurde durch Restriktionsverdau überprüft. Weiter wurde die korrekte Sequenz und Orientierung des SV40 ori-Fragments in pGS151 und pGS177 durch Sequenzanalyse bestätigt. Die adenoviralen Sequenzen in pSTK146, pGS119 und pGS122 wurden mittels Sequenzanalyse ermittelt und stimmten vollständig mit der Ad5 Wildtyp-Sequenz überein.

### b. Überprüfung der transienten Expression

Plasmide pSTK146, pGS119 und pGS122 wurden in HeLa-Zellen transfiziert und die Expression der E1A- und E1B-Proteine mittels Western Blot unter Verwendung von monoklonalen Antikörpern analysiert (Merck Bioscience). Plasmide pGS158, pGS159, pGS161 wurden in HEK293-Zellen transfiziert und die Expression des T-Antigens mittels Western Blot und eines monoklonalen Antikörpers (Abcam, Cambridge, UK) nachgewiesen. Plasmide pGS 116 und pGS 151 wurden in CAP-Zellen transfiziert und die Expression und Sekretion von humanem Alphal-Antitrypsin (hAAT) in den Kulturüberstand mittels ELISA (siehe 6.) nachgewiesen.

Ebenso wurden Plasmide pGS127 und pGS177 in CAP-Zellen transfiziert und die Expression von humanem Epo mittels ELISA nachgewiesen (siehe 6.).

### 3. Kultivierung von Zellen

### a. Zelllinien

Transformierte Amniozyten- (CAP- und CAP-T-) Zellen wurden in 293SFMII Medium (Invitrogen #11686-029), 0,5% Antimykotikum/Antibiotikum (Invitrogen #15240-062), 4 mM L-Glutamin (Invitrogen #25030-024) bei 37°C, 95% Luftfeuchtigkeit, 8% CO₂ kultiviert. Das Kultivierungsmedium von CAP-T-Zellen enthielt zusätzlich noch 5 µg/ml Blasticidin (Invitrogen # R210-01). Die Zellen wurden üblicherweise mit einer Startdichte von 2-4 x 10⁵ Zellen/ml in einem Volumen von 12 ml in einer Schüttelflasche angeimpft und im Schüttelinkubator bei 100 rpm für 3-4 Tage kultiviert. Bei einer Dichte von 1-2x10⁶ Zellen/ml wurden die Zellen durch Zentrifugation geerntet und mit oben beschriebener Startdichte in frischem Medium weiter kultiviert.

HEK293-, HEK293-T- (ATCC# CRL-11268) und HeLa Zellen wurden in Dulbecco's Modified Eagles Medium (Advanced D-MEM, Invitrogen #12491-015) mit 10% fötalem Kälberserum adherent in Zellkulturschalen kultiviert. HEK293-T-Zellen wurden schrittweise an serum-freies Suspensionswachstum in 293-SFMII Medium adaptiert und in Schüttelflaschen bei 100 rpm, 37°C, 95% Luftfeuchtigkeit und 8% CO₂ kultiviert.

### b. Primäre Amniozyten

Primäre Amniozyten wurden, entsprechenden Routinemethoden folgend, im Rahmen einer Fruchtwasserpunktion gewonnen. Von dieser Punktion wurden 1-2 ml mit 5 ml Ham's F10 Medium (Invitrogen #31550-023), 10% fötalem Kälberserum, 2% Ultroser G (CytoGen GmbH), 1x Antibiotikum/Antimycotikum (Invitrogen #15240-062), bei 37°C, 95% Luftfuchtigkeit und 5% CO₂ in 6-cm-Primaria Zellkulturschalen (Falcon) kultiviert. Nach 4-6 Tagen begannen die Amniozyten, adhärent zu werden, und es wurden 3 ml frisches Medium plus Zusätze (siehe vorhergehenden Satz) zugegeben. Waren die Zellen vollständig adhärent, wurde das Medium abgenommen und durch 5 ml frisches Medium plus Zusätze ersetzt. Für die weiteren Passagen wurden die konfluenten Zellen mit PBS gewaschen, mit Trypsin (TrypleSelect, Invitrogen #12563011) abgelöst und in 10 bzw. 25 ml frischem Medium plus Zusätze auf 10-cm- bzw.15-cm-Schalen übertragen.

### 4. Transformation von primären Amniozyten

### a. Transfektion

Kultivierte primäre Amnioyzten (siehe 3b) wurden durch Transfektion jeweils mit den Plasmiden pSTK146, pGS119 oder pGS122 transformiert. Vorab wurden die jeweiligen Plasmide mittels Verdau mit geeigneten Restriktionsenzymen (pSTK146, pGS119: ScaI; pGS122: PmeI) linearisiert. Vor der Transfektion wurden die Amniozyten schrittweise an Opti-Pro Medium (Invitrogen #12309-019) mit 2% Ultroser adaptiert. Dazu wurden die Zellen immer nach jeweils 2-3 Tagen mit frischem Ham's F10 Medium (mit Zusätzen siehe 3 b) plus Opti-Pro Medium (mit 2 % Ultroser) im Verhältnis 75:25%, 50:50%, 25:75% und 0:100% versetzt. Für die Transfektion wurden die Zellen einer ca. 80% konfluenten 15-cm-Schale auf sechs 6-cm-Schalen verteilt, was einer Zellzahl von 5-7x10⁵ Zellen pro Schale entsprach. Am nächsten Tag wurden die Zellen auf 5 Schalen mit je 2 µg linearisiertem pSTK146, pGS119 oder pGS122 unter Verwendung des Transfektionsreagenz Effectene (Qiagen) nach Angaben des Herstellers transfiziert. Eine Schale wurde untransfiziert als Kontrolle weiter kultiviert. Am nächsten Tag wurden die Zellen mit PBS gewaschen, mit TrypleSelect abgelöst und auf je eine 15-cm-Schale übertragen. Die Zellen wurden für weitere 10-15 Tage kultiviert, wobei alle 3-4 Tage das Medium durch frisches Medium ersetzt wurde. Während dieser Zeit wurde der Zusatz von Ultroser auf 1% gesenkt. Nach ca. 10-15 Tagen waren die Zellen konfluent und wurden auf je zwei 15-cm-Schalen, wie oben beschrieben, umgesetzt.

### b. Isolierung transformierter Zellklone

Wenige Wochen nach der Transfektion waren bei allen Transfektionen klonale Zellinseln zu beobachten, die sich morphologisch deutlich von den nicht-transformierten Amniozyten unterschieden. Diese Zellinseln wurden gepickt und auf 24-well-Schalen übertragen (entspricht Passage 1). Die Zellen wurden weiter vermehrt und erst auf 6-cm-, danach auf 15-cm-Schalen übertragen. Die Expression der E1-Proteine in den jeweiligen klonalen Zelllinien wurden in Western Blot Analysen unter Verwendung monoklonaler Antikörper nachgewiesen (siehe 2b).

Die Herstellung von T-Antigen-exprimierenden Zelllinien basierend auf transformierten Amniozytenzelllinien ist nachfolgend exemplarisch für eine, durch Transfektion mit pGS119 entstandene Zelllinie (nachfolgend CAP-Zelllinie genannt) beschrieben. Nach der Isolierung und Expansion der klonalen Zellinseln wurden aus den Zellklonen durch Einzelzellklonierung im "limited-dilution-Verfahren" genetisch einheitliche Linien hergestellt. Zusammengefasst wurden dazu eine Zelle des zu klonierenden Klons in eine Vertiefung einer 96-well Platte plattiert und mikroskopisch im Verlauf der kommenden Tage die tatsächliche Expansion von nur einer Zelle kontrolliert. Aus einzelnen Zellen entstandene Linien wurden schrittweise bis zu 15-cm Schalen expandiert. Durch schrittweise Verdünnung des Kultivierungsmediums Opti-Pro/1% Ultroser mit 293SFMII Medium wurden die Zellen an Wachstum in Suspension in serum-freiem Medium adaptiert. Die einzelnen Zelllinien wurden auf stabile und transiente Proteinexpression und hohe Wachstumsdichte untersucht, ein Klon mit den besten Eigenschaften wurde ausgewählt und nachfolgend weiterverwendet.

### 5. Herstellung von T-Antigen-exprimierenden Zellpools

Je 1x10⁷ CAP-Zellen (entstanden durch Transfektion von primären Amniozyten mit Plasmid pGS119, adaptiert an Suspensionswachstum im serum-freiem Medium) wurden mit je 5 µg linearisierter pGS158-, pGS159- und pGS161-Plasmid-DNA transfiziert und im Schüttelkolben unter den oben beschriebenen Bedingungen kultiviert. Zur Selektion stabil transfizierter Zellen wurden 48h nach Transfektion 5µg/ml Blasticidin zugegeben und die Zellen weiter kultiviert, bis nach ca. 3-4 Wochen stabil wachsende Zellpools entstanden. Die Zellpools erhielten die Bezeichnung Z582 (Transfektion mit pGS158, T-Antigen exprimiert vom CAG-Promotor), Z583 (Transfektion mit pGS159, T-Antigen exprimiert vom RSV-Promotor) und Z597 (Transfektion mit pGS161, T-Antigen exprimiert vom CMV-Promotor). Da unbekannt ist, ob eine erhöhte T-Antigen-Konzentration eventuell toxisch für CAP-Zellen ist, wurde versucht, T-Antigen mit Hilfe unterschiedlich starker Promotoren zu exprimieren. Es konnte gezeigt werden, dass die Expression eines Referenzproteins in CAP-Zellen am höchsten ausfällt bei der Verwendung des CMV-Promotors, gering schwächer mit dem CAG-Promotor und deutlich schwächer mit dem RSV-Promotor. Mit allen drei Promotoren konnten stabil wachsende Zellpools generiert werden und alle drei Zellpools exprimieren intrazelluläres T-Antigen.

### 6. Transiente Proteinexpression in CAP- und CAP-T-Zellen

Das hier verwendete 359-bp ori-Fragment enthält gegenüber dem 63 bp langen minimalen ori zusätzlich zu dieser "Core"-Sequenz noch die 21-bp- und 72-bp-Wiederholungssequenzen (SEQ ID NO:11). Diese beiden Wiederholungssequenzen sind zwar hauptsächlich wichtig für die Funktion des mit dem ori überlappenden Promotors, es gibt jedoch Hinweise, dass sie auch die SV40-DNA-Replikation verstärken (Chandrasekharappa and Subramanian, J. Virol. 61, 2973-2980, 1987).

Um zu testen, ob die Konzentration an T-Antigen in der Zelle einen Einfluss auf die Expression eines Referenzproteins hat, wurden drei Zellpools Z582, Z583 und Z597 getestet, die T-Antigen unter unterschiedlich starken Promotoren exprimieren, und mit der transienten Expression in CAP-Zellen, die kein T-Antigen exprimieren, verglichen. Dazu wurden je 1x10⁷ Zellen mit Hilfe der Nucleofector Technologie (Amaxa/Lonza, Programm X-001, Puffer V) mit dem zirkulären Plasmid pGS 151 transfiziert und in einem Startvolumen von 12 ml kultiviert. Drei und sechs Tage nach Transfektion wurde das Medium ausgetauscht, wobei am Tag 6 auch das Volumen auf 15 ml erhöht wurde. Ab dem dritten bis einschließlich am siebten Tag nach Transfektion und am neunten Tag nach Transfektion wurde jeweils ein Aliquot abgenommen, die Zellzahl bestimmt und die Expression von hAAT mit Hilfe der ELISA (Enzyme-linked Immunosorbent Assay)-Methode unter Verwendung von polyklonalen Anti-hAAT-Antikörpern (ungekoppelt und HRP-gekoppelt; ICN Biomedicals) bestimmt. Als Kontrolle wurde aus humanem Plasma gereinigtes hAAT (ICN Biomedicals) verwendet.

Das Ergebnis dieses Experiments ist graphisch in Fig. 3 dargestellt. In allen CAP-T-Zellpools wurde eine höhere transiente Expression erzielt verglichen mit den CAP-Zellen. Die transiente Expression in Z582 liegt 8-fach, in Z583 25-fach und in Z597 70-fach über der von CAP Zellen. Auch ein zweiter CAP-T-Zellpool mit T-Antigen-Expression vom CMV-Promotor aus ergab eine vergleichbar hohe Expression wie mit Z597.

Diese Daten zeigen, dass sowohl die permanente T-Antigen-Expression in CAP-Zellen als auch die Höhe der T-Antigen-Expression einen Einfluss auf die Höhe der transienten Expression hat.

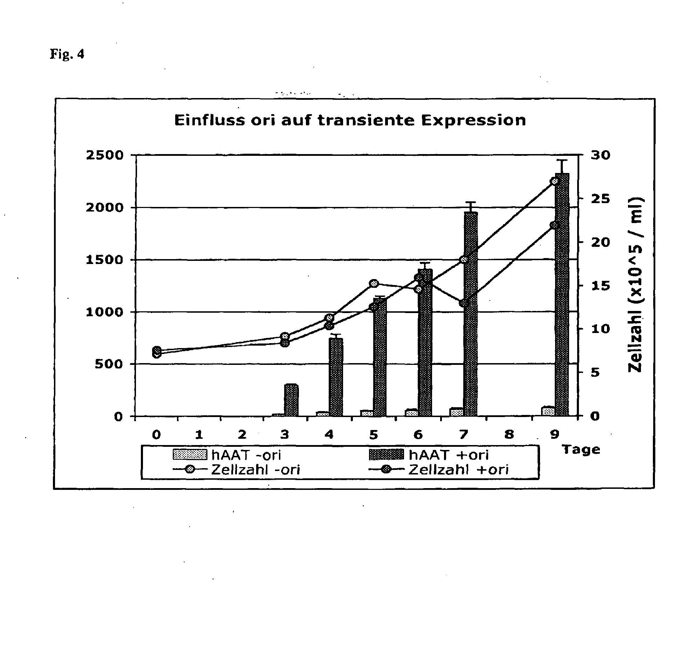

In einem weiteren Experiment wurden die transienten Expressionshöhen von hAAT in dem Zellpool Z597 nach transienter Transfektion des Plasmids pGS116 bzw. pGS151 bestimmt. Diese beiden Plasmide unterscheiden sich nur durch die Anwesenheit des SV40-ori-Fragmentes in pGS151. Die Transfektion und quantitative Analyse von hAAT wurden wie oben beschrieben durchgeführt, wobei sowohl die Höhe der Expression von hAAT als auch die Entwicklung der Lebendzellzahl über den Versuchszeitraum von 9 Tagen bestimmt wurde. Das Ergebnis dieses Versuchs ist in Fig. 4 graphisch dargestellt. Die Anwesenheit des SV40-ori-Fragments im Expressionsplasmid führt zu einer 30-fach erhöhten transienten Expression. Insgesamt konnte durch Transfektion von 1x10⁷ CAP-T Zellen innerhalb von 9 Tagen 2,5 mg hAAT in 40 ml Volumen exprimiert werden, was einer Expressionsleistung von ca. 60 mg/L und bis zu 40 pg/Zelle/Tag entspricht. Etwa 3 Tage nach Transfektion setzt das Zellwachstum ein, die Vitalität der Zellen bleibt weiter über den gesamten Versuchszeitraum über 80%.

Um zu zeigen, dass diese transiente Expressionsleistung nicht spezifisch ist für hAAT, wurde ein weiteres hoch-glykosyliertes Protein Erythropoietin (Epo) in CAP-T Zellen transient exprimiert. Wie für hAAT beschrieben, wurden 1x10⁷ CAP-T Zellen aus dem Z597-Zellpool mit den Plasmiden pGS 177 (enthält die Epo-Expressionskassette und das SV40-ori-Fragment) transfiziert und Epo im Zellkulturüberstand mittels ELISA (R&D Systems, Quantikine IVD, Human Epo Immunoassay, DEP00) quantifiziert. Über einen Versuchszeitraum von 7 Tagen konnten 0,73 mg Epo exprimiert werden bei einer Expressionsleistung von 32 mg/L.

### 7. Vergleich mit transienter Expression in anderen Zellsystemen

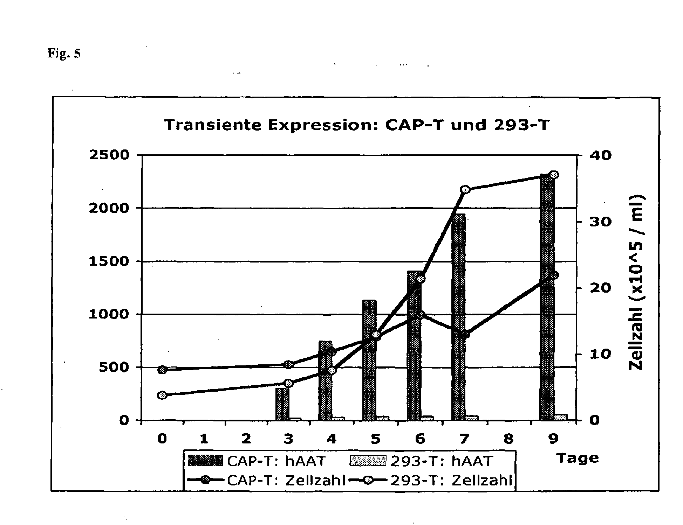

Eine bereits früher beschriebene humane Zelllinie, die sog. HEK293-T-Zelllinie, exprimiert stabil das SV40 T-Antigen und basiert auf der humanen, Adenovirus-transformierten HEK293-Zelllinie (DuBridge et al., Mol. Cell. Biol. 7, 379-387, 1987). Vergleichbar mit Z597, wurden 1x10⁷ HEK293-T-Zellen (serum-freies Medium, Suspensionskultur) mit 5µg zirkulärem Plasmid pGS151 mit Hilfe der Amaxa-Nucleofektor-Technologie nach Angaben des Herstellers (Programm X-001, Puffer V) transfiziert und kultiviert. Das Ergebnis dieses Experiments ist in Fig. 5 graphisch dargestellt. Obwohl die 293-T-Zellzahl am Tag 9 deutlich höher ist als mit CAP-T, ist die transiente Expression in CAP-T gegenüber 293-T ca. 40-fach erhöht.

### 8. Replikationsassay

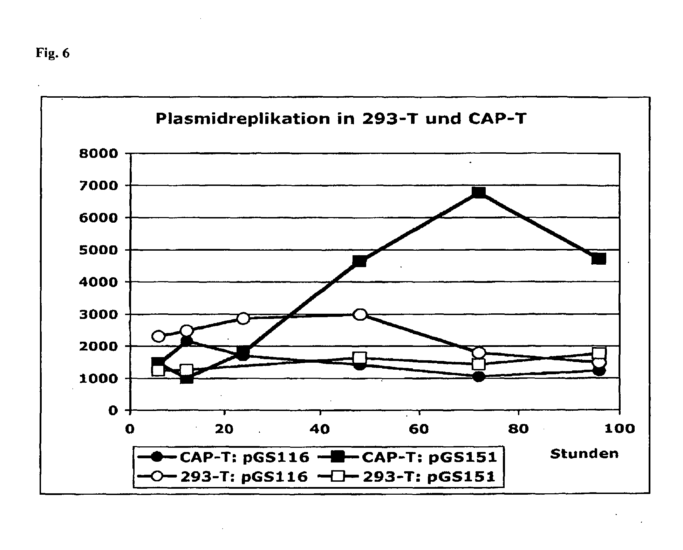

In einem Replikationsassy sollte gezeigt werden, ob die Expression von T-Antigen in CAP-T-Zellen zu einer erhöhten Kopienzahl des ori-enthaltenden Expressionsplasmids führt und somit die deutlich erhöhte transiente Proteinexpression erklärt. Dazu wurden Z597- bzw. HEK293-T-Zellen mit den Plasmiden pGS116 bzw. pGS151 transfiziert und wie oben beschrieben kultiviert. Nach 6, 12, 24, 48, 72 und 96 Stunden wurden je 1x10⁵ Zellen entnommen, abzentrifugiert, in PBS aufgenommen und durch Zugabe von gleichem Volumen 0,8 N NaOH lysiert. Die Zelllysate wurden in einer SlotBlot-Apparatur auf eine positiv geladene Nylonmembran (GE Healthcare, Hybond-N+) geblottet. Zur Kontrolle wurden steigende Plasmidmengen pGS116 und pGS151 zu 1x10⁵ Z597-Zellen gegeben, wie oben beschrieben lysiert, und geblottet. Dieser Standard entsprach 1000, 2500, 5000, 10000 und 15000 Kopien pro Zelle. Die DNA wurde durch 30-minütige Inkubation der Membran bei 120°C fixiert und mit Hilfe einer nicht-radioaktiv markierten PCR-Sonde aus der hAAT-cDNA nach Angaben des Herstellers (AlkPhos Direct Labelling and Detection System, GE Healthcare, RPN 3680 und 3682) sichtbar gemacht. Die Kopienzahl in pGS116- und pGS151-transfizierten Zellen wurde anhand der bekannten Konzentration des Standardplasmids quantifiziert. Das Ergebnis dieses Replikationsassays ist in Fig. 6 graphisch dargestellt. Wie erwartet, ist nur pGS151, nicht aber pGS116 in CAP-T Z597 repliziert. Bei gleich bleibender Zellzahl steigt die Kopienzahl von pGS151 von ca. 1500 Kopien/Zelle 6h nach Transfektion auf fast 7000 Kopien/Zelle 72h nach Transfektion an. Demgegenüber bleibt die Kopienzahl von pGS 151 in HEK293-T über 96h konstant. Da sich die 293-T-Zellzahl in diesem Zeitraum verdoppelt hat, kann von einer schwachen Replikation von pGS151 in diesen Zellen ausgegangen werden, sie liegt jedoch deutlich unter der Replikationsrate von Z597.

Der Nachweis der Expression von T-Antigen in den Amniozytenzelllinien und HEK293-T-Zellen wurde durch Western Blot-Analyse geführt. Von den drei CAP-T-Zellpools und HEK293-T Zellen wurden je 1x10⁶ Zellen in 50 µl 50 mM Tris/HCL pH8, 140 mM NaCL, 0.5% NP40, 4 mM EDTA, 2 mM EGTA, 0.5 mM PMSF, 5% Glycerol aufgenommen und für 30min auf Eis inkubiert. Das Proteingemisch wurde für 10 min bei 13 000 rpm abzentrifugiert und die Proteinkonzentration im Überstand mit Hilfe eines Proteinbestimmungskits (Coomassie, Bradford, Thermo Life Science# 23200) bestimmt. Auf einem 12%igen SDS-Polyacrylamidgel wurden je 10 µg Protein aufgetrennt, auf eine Nitrozellulosemembran (Hybond ECL, Amersham Pharmacia Biotech) transferiert und mit Hilfe eines T-Antigen-spezifischen Antikörpers (Abcam, Anti-SV40 T-Antigen ab16879) sichtbar gemacht. Mit diesem Experiment konnte gezeigt werden, dass in Z597 mehr T-Antigen exprimiert wird als in den beiden anderen Pools und als in HEK-293-T-Zellen.

### 9. Transfektion mit Polyethylenimin

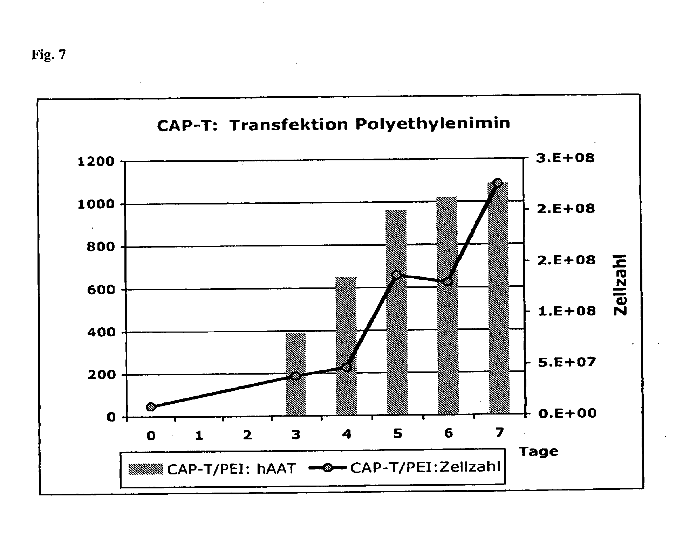

Da die oben beschriebene Transfektionsmethode nur bedingt skalierbar ist, wurde ein weiteres Transfektionsreagenz, Polyethylenimin (PEI, Polysciences, #23966), getestet, das vor allem für Transfektionen in großem Maßstab beschrieben wurde. Lineares PEI (MW=25.000) wurde nach Angaben des Herstellers mit einer Konzentration von 1 mg/ml gelöst und im Verhältnis DNA:PEI=1:3 eingesetzt. Zur Transfektion wurden 10 µg pGS151 mit 30 µg PEI gemischt, 10 min bei Raumtemperatur inkubiert und zu 1x10⁷ CAP-T Z597-Zellen in 6 ml FreeStyle-Medium (Invitrogen #12338-018) gegeben. Nach 5h wurden 6 ml 293-SFMII Medium zugegeben und die Zellen über 7 Tage inkubiert. Drei Tage nach Transfektion wurde das Medium durch 293-SFMII ausgetauscht und aufgrund des starken Wachstums der Zellen wurde das Volumen auf 30 ml erhöht. Das Ergebnis dieses Experiments ist in der Fig. 7 dargestellt. Durch die Transfektion mit PEI wurde eine hohe transiente Proteinexpression in CAP-T erreicht. Allerdings lag die maximale Proteinausbeute ca. 2-fach unter der mit Nukleofektion erzielten Expression. Auffallend ist, dass die Zellen nach Transfektion mit PEI deutlich schneller und stärker wachsen und nach 7 Tagen die ca. 10-fache Zellzahl im Vergleich zu Nukleofektion erreicht wird.

## Patentansprüche

1. Verfahren zur Herstellung einer permanenten humanen Amniozyten-Zelllinie umfassend:
a) Transfizieren primärer humaner Amniozyten-Zellen mit einem Nukleinsäuremolekül umfassend eine Nukleinsäuresequenz kodierend für die adenoviralen Genprodukte E1A und E1B und
b) anschließendes Transfizieren der in Schritt a) erhaltenen permanenten humanen Amniozyten-Zellinie mit einem Nukleinsäuremolekül umfassend eine Nukleinsäuresequenz kodierend für das große T-Antigen von SV40 oder das Epstein-Barr-Virus (EBV) Nuclear Antigen-1 (EBNA-1).

2. Das Verfahren nach Anspruch 1, wobei die Nukleinsäuresequenz kodierend für die adenoviralen Genfunktionen E1A und E1B von einem humanen Adenovirus stammt, insbesondere vom humanen Adenovirus Serotyp 5.

3. Das Verfahren nach Anspruch 2, wobei die Nukleinsäuresequenz kodierend für die adenoviralen Genprodukte E1A und E1B die Nukleotide 1 bis 4344, 505 bis 3522 oder die Nukleotide 505 bis 4079 des humanen Adenovirus Serotyp 5 umfasst.

4. Das Verfahren nach Anspruch 1, wobei die Nukleinsäuresequenz kodierend für das große T-Antigen von SV40 ferner die Nukleinsäuresequenz für einen Promotor ausgewählt aus der Gruppe CMV-Promotor, CAG-Promotor und RSV-Promotor, die Nukleinsäuresequenz für SV40 SD/SA (intron) und die Nukleinsäuresequenz für SV40 polyA umfasst, und die Nukleinsäuresequenz für das Epstein-Barr-Virus (EBV) Nuclear Antigen-1 (EBNA-1) ferner die Nukleinsäuresequenz für einen Promotor ausgewählt aus der Gruppe CMV-Promotor, CAG-Promotor und RSV-Promotor, die Nukleinsäuresequenz für SV40 SD/SA (intron) und die Nukleinsäuresequenz für SV40 polyA umfasst.

5. Das Verfahren nach einem der vorhergehenden Ansprüche, wobei anstelle des Schritts a) bereits mit einem Nukleinsäuremolekül umfassend eine Nukleinsäuresequenz kodierend für die adenoviralen genprodukte E1A und E1B immortalisierte humane Amniozyten-Zellen verwendet werden.

## Claims

1. A method for the production of a permanent human amniocytic cell line comprising:
a) transfecting primary human amniocytic cells with a nucleic acid molecule comprising a nucleic acid sequence encoding the adenoviral gene products E1A and E1B and
b) subsequently transfecting the permanent human amniocytic cell line obtained in step a) with a nucleic acid molecule comprising a nucleic acid sequence encoding the SV40 large T-antigen or the Epstein-Barr virus (EBV) nuclear antigen 1 (EBNA-1).

2. The method according to claim 1, wherein the nucleic acid sequence encoding the adenoviral gene functions E1A and E1B is derived from a human adenovirus, in particular the human adenovirus serotype 5.

3. The method according to claim 2, wherein the nucleic acid sequence encoding the adenoviral gene products E1A and E1B comprises the nucleotides 1 to 4344, 505 to 3522, or the nucleotides 505 to 4079 of the human adenovirus serotype 5.

4. The method according to claim 1, wherein the nucleic acid sequence encoding the SV40 large T-antigen further comprises the nucleic acid sequence for a promoter selected from the group of CMV promoter, CAG promoter and RSV promoter, the nucleic acid sequence for SV40 SD/SA (intron) and the nucleic acid sequence for SV40 polyA, and the nucleic acid sequence for the Epstein-Barr virus (EBV) nuclear antigen 1 (EBNA-1) further comprises the nucleic acid sequence for a promoter selected from the group of CMV promoter, CAG promoter und RSV promoter, the nucleic acid sequence for SV40 SD/SA (intron) and the nucleic acid sequence for SV40 polyA.

5. The method according to any one of the preceding claims, wherein instead of step a) human amniocytic cell lines already immortalized with a nucleic acid molecule, comprising a nucleic acid sequence encoding the adenoviral gene products E1A and E1B, are used.

## Revendications

1. Procédé de production d'une lignée cellulaire permanente d'amniocytes humains comprenant :
a) la transfection de cellules d'amniocytes humains primaires avec une molécule d'acide nucléique comprenant une séquence d'acide nucléique codant pour les produits de gène adénoviraux E1A et E1B et
b) la transfection consécutive de la lignée cellulaire permanente d'amniocytes humains obtenue à l'étape a) avec une molécule d'acide nucléique comprenant une séquence d'acide nucléique codant pour le grand antigène T de SV40 ou l'antigène 1 nucléaire du virus d'Epstein-Barr (EBV) (EBNA-1).

2. Procédé selon la revendication 1, dans lequel la séquence d'acide nucléique codant pour les fonctions de gène adénovirales E1A et E1B provient d'un adénovirus humain, en particulier d'un adénovirus humain de sérotype 5.

3. Procédé selon la revendication 2, dans lequel la séquence d'acide nucléique codant pour les produits de gène adénoviraux E1A et E1B comprend les nucléotides 1 à 4344, 505 à 3522 ou les nucléotides 505 à 4079 de l'adénovirus humain de sérotype 5.

4. Procédé selon la revendication 1, dans lequel la séquence d'acide nucléique codant pour le grand antigène T de SV40 comprend en outre la séquence d'acide nucléique d'un promoteur choisi dans le groupe comprenant le promoteur du CMV, le promoteur CAG et le promoteur RSV, la séquence d'acide nucléique pour SV40 SD/SA (intron) et la séquence d'acide nucléique pour SV40 polyA et la séquence d'acide nucléique de l'antigène 1 nucléaire du virus d'Epstein-Barr (EBV) (EBNA-1) comprend en outre la séquence d'acide nucléique d'un promoteur choisi dans le groupe comprenant le promoteur du CMV, le promoteur CAG et le promoteur RSV, la séquence d'acide nucléique de SV40 SD/SA (intron) et la séquence d'acide nucléique de SV40 polyA.

5. Procédé selon l'une des revendications précédentes, dans lequel, à la place de l'étape a), on utilise des cellules d'amniocytes humains déjà immortalisées avec une molécule d'acide nucléique comprenant une séquence d'acide nucléique codant pour les produits de gène adénoviraux E1A et E1B.
